# EUROPEAN PATENT SPECIFICATION

(11) **EP 0 379 057 B1**
(45) Date of publication and mention of the grant of the patent: **06.12.2000**
(21) Application number: 90100491.1
(22) Date of filing: 11.01.1990
(51) Int. Cl.: C08G 59/00, C08G 59/20

(54) **Mesogenic epoxy compounds**
Mesogene Epoxydverbindungen
Composés époxy mésogènes

(30) Priority: 17.01.1989 US 298431
(43) Date of publication of application: 25.07.1990
(73) Proprietor: THE DOW CHEMICAL COMPANY, Midland, Michigan 48674 (US)
(72) Inventor: Earls, Jimmy D., Lake Jackson, Texas 77566 (US); Hefner, Robert E., Jr., Lake Jackson, Texas 77566 (US); Puckett, Paul M., Lake Jackson, Texas 77566 (US)
(74) Representative: Kremer, Robert A.M.

(56) References cited:
- EP-A- 0 172 012
- EP-A- 0 235 506
- EP-A- 0 252 359
- EP-A- 0 274 128
- EP-A- 0 287 233
- EP-A- 0 361 853
- US-A- 3 383 360
- CHEMICAL PATENTS INDEX, BASIC ABSTRACTS JOURNAL, Section A, week 8834, 19th October 1988, A0285, no. 88-237750/34, Derwent Publications Ltd., London, GB; & JP-A-63 169 619 (NIPPON TELEG. & TELEPH. (TORA), 13-07-1988

## Description

The present invention pertains to epoxy resins containing mesogenic or rodlike moietie(s), curable compositions and cured compositions thereof.

Epoxy resins are useful in many applications such as coatings, laminates, composites, adhesives, and castings. In each of these applications, it is desirable to have epoxy resins with an improvement in any one or more of their physical and/or thermal and/or chemical resistant properties.

The present invention provides a method for improving one or more of these properties by incorporating into the polymer chain of the epoxy resin one or more mesogenic or rodlike structure(s). These epoxy resins exhibit ordering of the molecular chains in the melt phase and/or in the advanced compositions thereof. This morphology is susceptible to flow induced orientation during processing which can result in enhanced unidirectional, mechanical properties. This is not possible to any great extent with conventional epoxy resins.

In contrast to other types of mesogenic polymers, which are primarily thermoplastics, the epoxy resins containing mesogenic or rodlike moieties provide an advantage in that final chain extension and/or crosslinking occurs during the curing stages of the fabricated part. This permits new systems which process at much lower temperatures.

The mesogenic or rodlike type structures incorporated into the chain provide the improvement in one or more of the properties. The property improvements obtained with epoxy resins of this type can be unidirectionally enhanced by electric or magnetic fields or by shear stresses applied during processing and/or curing.

EP - A 252 359 describes networks prepared from liquid crystal units, and more particularity derived from p-epoxy propoxy phenyl p-epoxy propoxy benzoate.

US -A- 3 383 360 discloses incidently the diglycidyl ether of p-azophenol without any indication of a possible liquid crystal property.

EP -A - 235 506 relates to polyoxyalkylene polymers exhibiting non linear optical response having a mesogenic side chain.

One aspect of the present invention pertains to epoxy resins containing one or more mesogenic or rodlike moieties represented by the following Formula I: 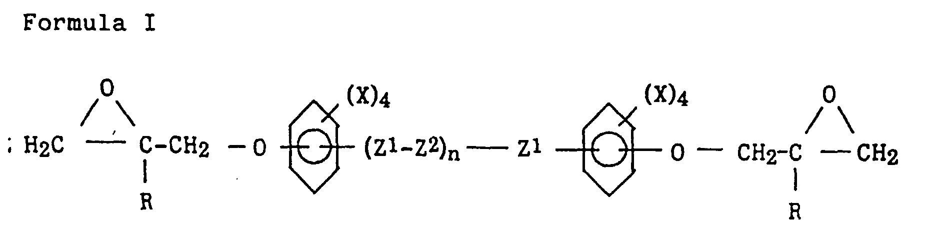 wherein at least about 80 percent of the -(Z¹-Z²)ₙ-Z¹-linkages and the glycidyl ether groups are in the para position with respect to each other; each R and R¹ is independently hydrogen or an aliphatic hydrocarbon group having from 1 to 4 carbon atoms; each X is independently hydrogen, a hydrocarbyl or hydrocarbyloxy group having suitably from 1 to 12, more suitably from 1 to 6, most suitably from 1 to 4 carbon atoms, a halogen atom (preferably chlorine or bromine), -NO₂, or -C≡N; each Z¹ is independently -CR¹=CR¹-, -CR¹=CR¹-CR¹=CR¹-, -CR¹=N-N=CR¹-, -CR¹=CR¹-CO-O-CH₂-, -CR1=CR¹-CO-O-CH₂-CH₂-, -CH₂-O-CO-CR¹=CR¹-, -CH₂-CH₂-O-CO-CR¹=CR¹-, -CR¹=CR¹=CO-O-, -O-CO-CR¹=CR¹-, -CO-NH-, -NH-CO-, -CO-NH-NH-CO-, -C≡C-, -C≡C-C≡C-, -CO-S-, -S-CO-, a direct single bond when n≧ 1, 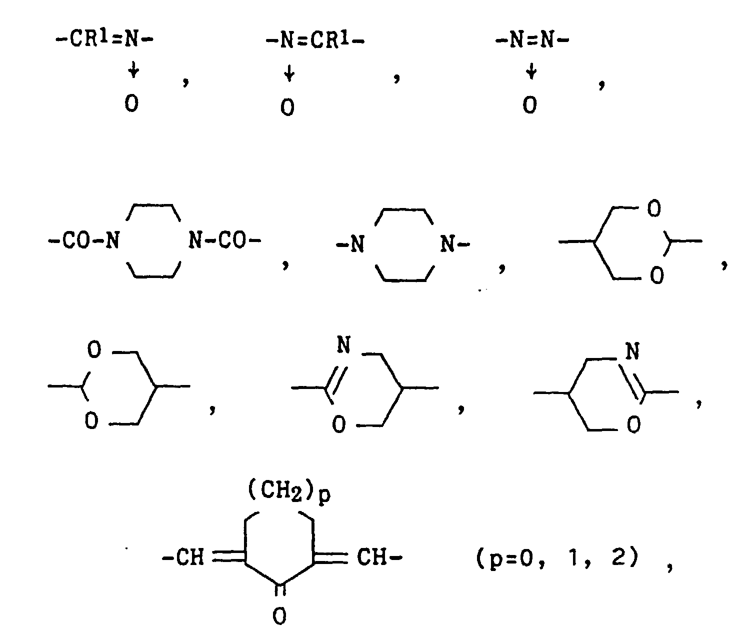 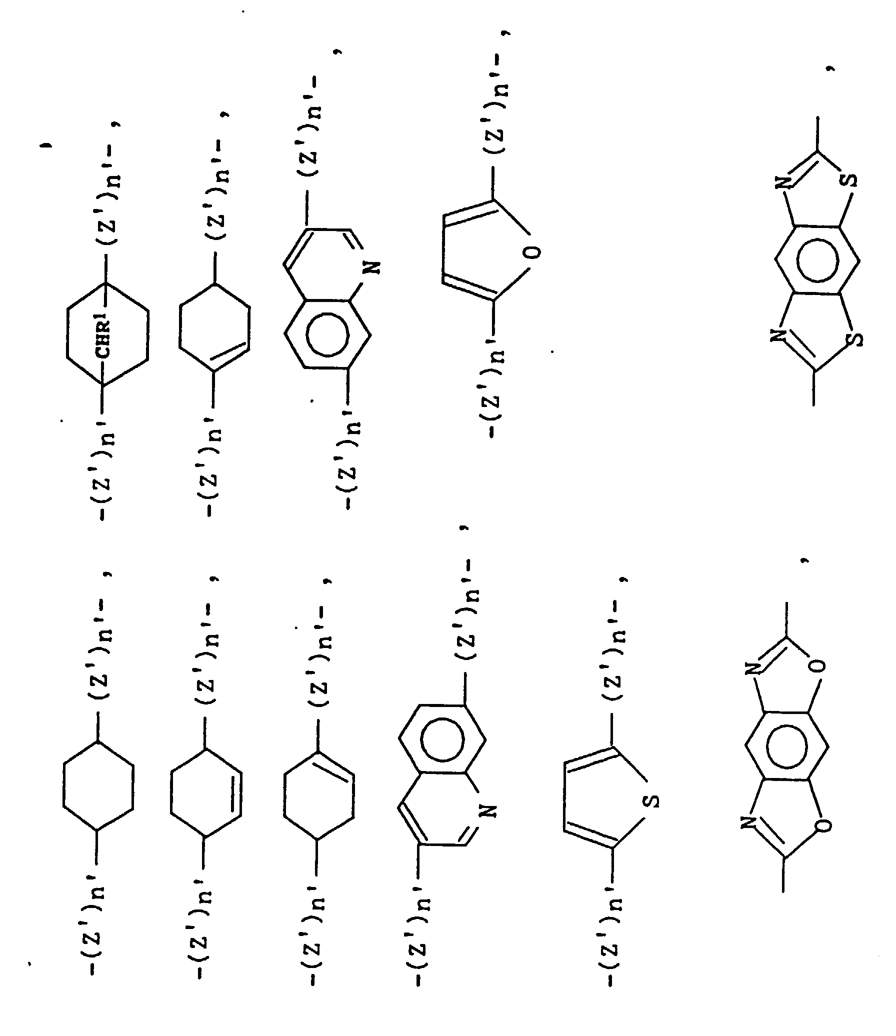 each Z¹ can also independently be 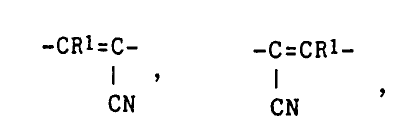 -CR¹=N-, -N=CR¹-, -CO-CR¹=CR¹-, -CR¹=CR¹-CO-, -CO-O-, and -O-CO- when Z² is not a benzene ring and n≠0; Z² is a group represented by a cyclic or bicyclic ring system containing from 5 to 12 carbon atoms and may be cycloaliphatic, polycycloaliphatic, aromatic or a combination thereof; n is 0 to 2; each Z' is independently a -CO-, -O-CO-, -CO-O-, -CO-NR¹-, or -NR¹-CO- group and each n' independently has a value of zero or one.

Another aspect of the present invention pertains to epoxy resins containing one or more mesogenic or rodlike moieties represented by the following Formula II: 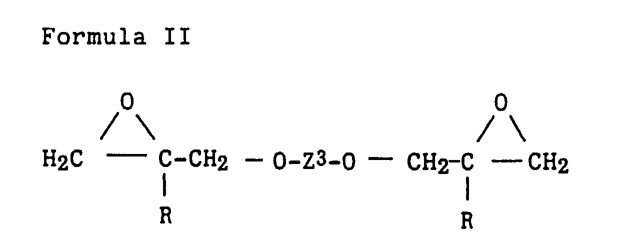 wherein Z³ is 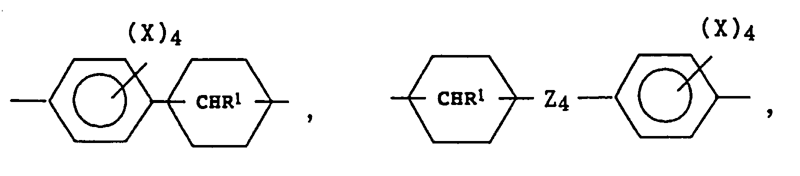 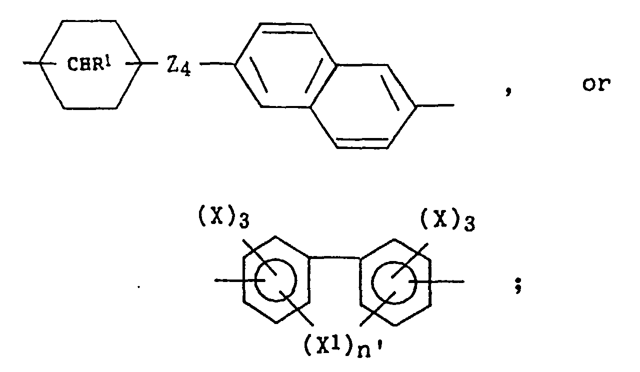 and Z⁴ is -CO-O-, -O-CO-, -NR¹-CO- or -CO-NR¹-; X¹ is a hydrocarbyl group having from 1 to 10, preferably from 1 to 4, carbon atoms which can contain one or more heteroatoms selected from N, O and S and may be saturated or unsaturated and X, R, R¹ and n are as previously defined.

Another aspect of the present invention pertains to advanced epoxy resin compositions prepared by reacting (A) one or more of the epoxy resins containing one or more mesogenic or rodlike moieties represented by Formulas I or II with (B) at least one compound having an average of more than one active hydrogen atom per molecule; wherein components (A) and (B) are employed in quantities which provide a ratio of active hydrogen atoms per epoxide group of from 0.01:1 to 0.95:1.

Another aspect of the present invention pertains to resin compositions prepared by advancement reaction of (A) one or more of the epoxy resins containing one or more mesogenic or rodlike moieties represented by Formulas I or II with (B) at least one compound having an average of more than one active hydrogen atom per molecule; wherein components (A) and (B) are employed in quantities which provide a ratio of active hydrogen atoms per epoxide group of from 0.96:1 to 1.05:1.

Another aspect of the present invention pertains to blends of (A) one or more of the epoxy resins or compounds containing one or more mesogenic or rodlike moieties represented by the aforementioned Formulas I or II, and (B) one or more polyepoxides represented by the following Formulas V, VI, VII, VIII, IX, X or XI: 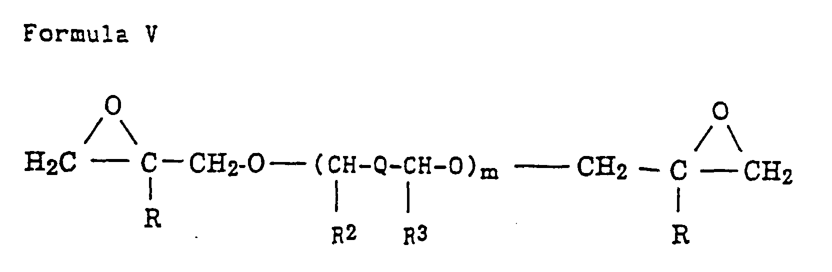 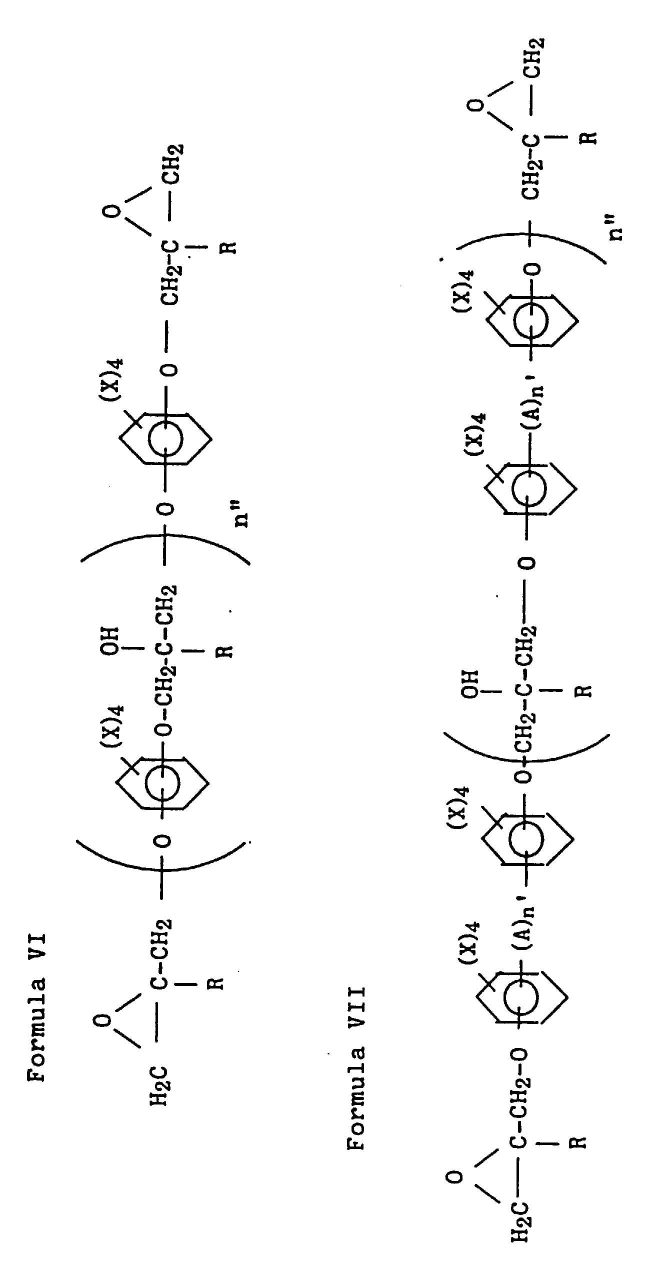 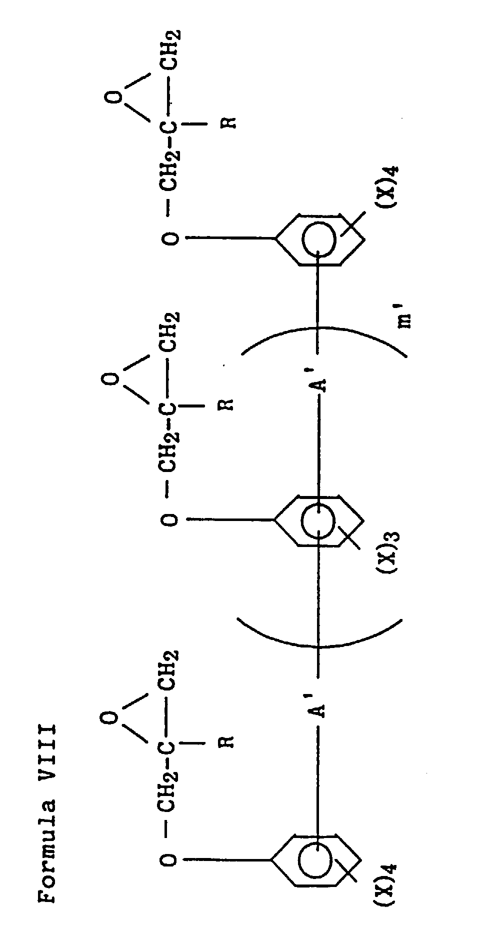 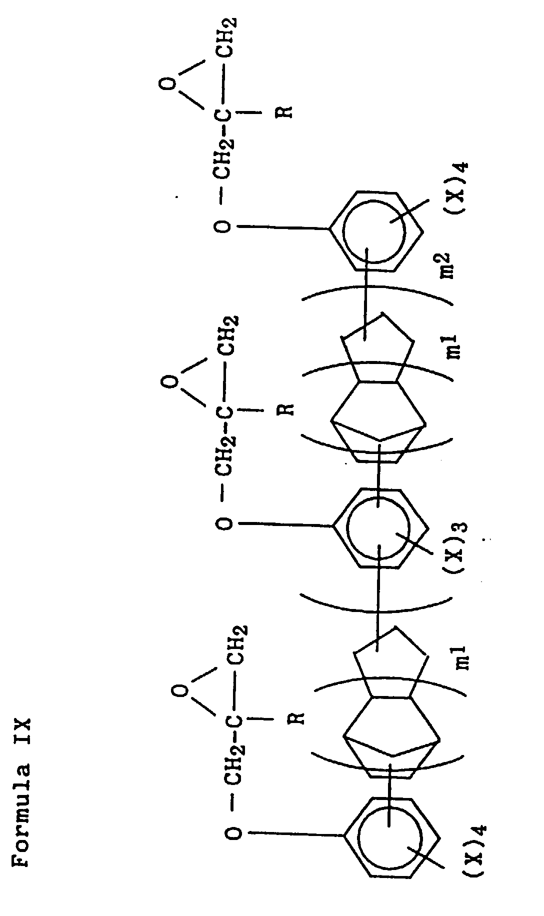 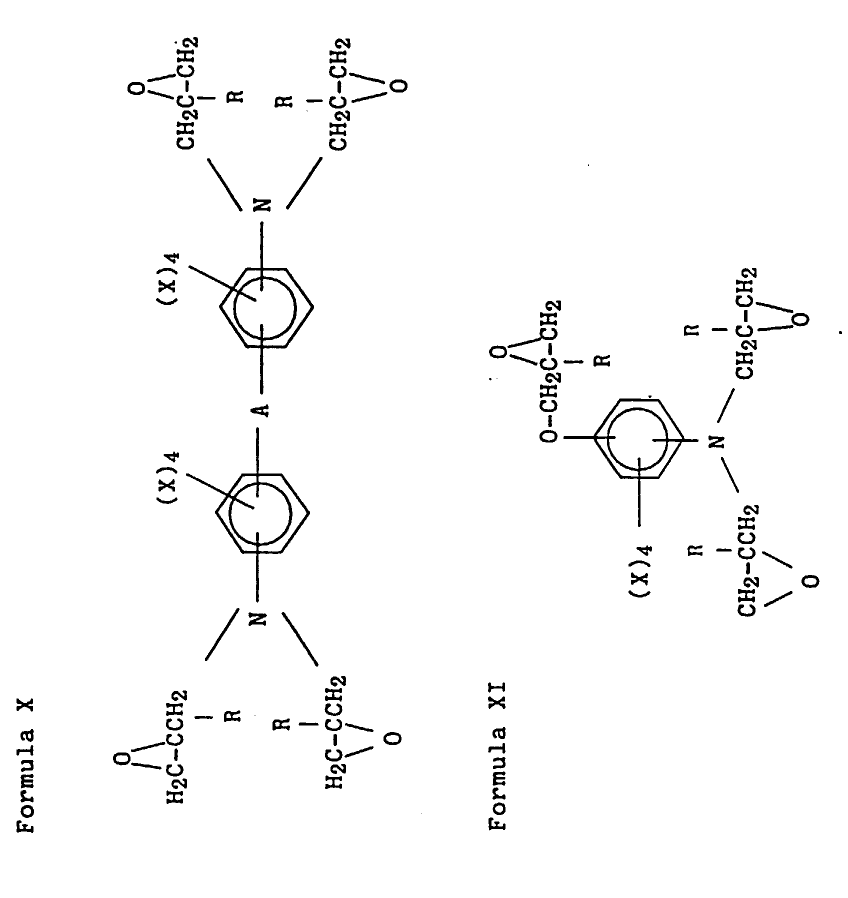 wherein each A is independently a divalent hydrocarbyl group having from 1 to 12, preferably from 1 to 6, more preferably from 1 to 3 carbon atoms, -O-, -S-, -S-S-, -SO-, -SO₂- or -CO-; each A' is independently a divalent hydrocarbon group having from 1 to 6, preferably from 1 to 3 carbon atoms; Q is a single bond, -CH₂-S-CH₂-, 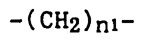 or 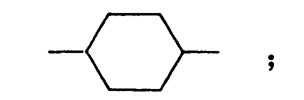 each R is independently hydrogen or an alkyl group having from 1 to 4 carbon atoms; each R² and R³ is independently hydrogen, a hydrocarbyl or halohydrocarbyl group having from 1 to 6, preferably from 1 to 3, more preferably from 1 to 2 carbon atoms; each X is independently hydrogen, a hydrocarbyl or hydrocarbyloxy group having from 1 to 12, preferably from 1 to 6, most preferably from 1 to 4 carbon atoms, a halogen atom, -NO₂ or -C≡N; m has a value from 1 to 10, preferably from 1 to 4, more preferably from 1 to 2; m' has an average value from 0.01 to 12, preferably from 1 to 6, more preferably from 1 to 3; m¹ has an average value from 1 to 12, preferably from 1 to 6, more preferably from 1 to 3; m² has a value from 1 to 12, preferably from 2 to 6, more preferably from 2 to 3; n' has a value of zero or 1; n'' has an average value from zero to 3, preferably from zero to 1.5, more preferably from zero to 0.5, and n¹ has an average value from 1 to 10 wherein component (A) is present in an amount suitably from 1 to 99, more suitably from 10 to 80, most suitably from 10 to 50 percent by weight based upon the combined weight of components (A) and (B) and component (B) is present in an amount suitably from 99 to 1, more suitably from 90 to 20, most suitably from 90 to 50 percent by weight based upon the combined weight of components (A) and (B).

A further aspect of the invention relates to blends comprising (A) one or more epoxy-containing resins or compounds containing one or more mesogenic or rodlike moieties of
advanced epoxy resin compositions prepared by reacting (A) one or more of the epoxy resins containing one or more mesogenic or rodlike moieties represented by Formulas I or II with (B) at least one compound having an average of more than one active hydrogen atom per molecule; wherein components (A) and (B) are employed in quantities which provide a ratio of active hydrogen atoms per epoxide group of from 0.01:1 to 0.95:1,
and (B) one or more polyepoxides not containing mesogenic or rodlike moieties; wherein component (A) is present in an amount of from 1 to 99 percent by weight of the combined weight of components (A) and (B) and component (B) is present in an amount of from 99 to 1 percent by weight of the combined weight of components (A) and (B).

Another aspect of the present invention pertains to curable compositions comprising at least one epoxy resin containing one or more mesogenic or rodlike moieties represented by the above Formula I and a curing amount of a suitable curing agent therefor.

Another aspect of the present invention pertains to curable compositions comprising at least one epoxy resin containing one or more mesogenic or rodlike moieties represented by the above Formula II and a curing amount of a suitable curing agent therefor.

Another aspect of the present invention pertains to curable compositions comprising (A) at least one of the epoxy resins containing one or more mesogenic or rodlike moieties represented by the above Formulas I or II, (B) at least one of the monoepoxide compounds containing one or more mesogenic or rodlike moieties selected among
monoepoxide compounds containing one or more mesogenic or rodlike moieties represented by the following Formula III: 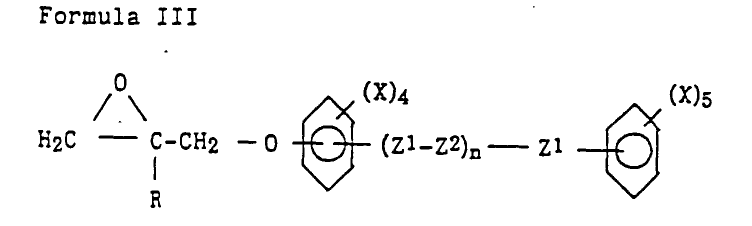 wherein at least about 80 percent of the -(Z¹-Z²)ₙ-Z¹-linkages and the glycidyl ether groups are in the para position with respect to each other; each R and R¹ is independently hydrogen or an aliphatic hydrocarbon group having from 1 to 4 carbon atoms; each X is independently hydrogen, a hydrocarbyl or hydrocarbyloxy group having suitably from 1 to 12, more suitably from 1 to 6, most suitably from 1 to 4, carbon atoms, a halogen atom (preferably chlorine or bromine), -NO₂, or -C≡N; each Z¹ is independently -CR¹=CR¹-, -CR¹=CR¹-CR¹=CR¹-, -CR¹=N-N=CR¹-, -CR¹=CR¹-CO-O-CH₂-, -CR¹=CR¹-CO-O-CH₂-CH₂-, -CH₂-O-CO-CR¹=CR¹-, -CH₂-CH₂-O-CO-CR¹=CR¹-, -CR¹=CR¹-CO-O-, -O-CO-CR¹=CR¹-, -N=N-, -CO-NH-, -NH-CO-, -CO-NH-NH-CO-, -C≡C-, -C≡C-C≡C-, -CO-S-, -S-CO-, -CR¹=N-, -N=CR¹-, -CO-O-, -O-CO-, a direct single bond when n≧1, 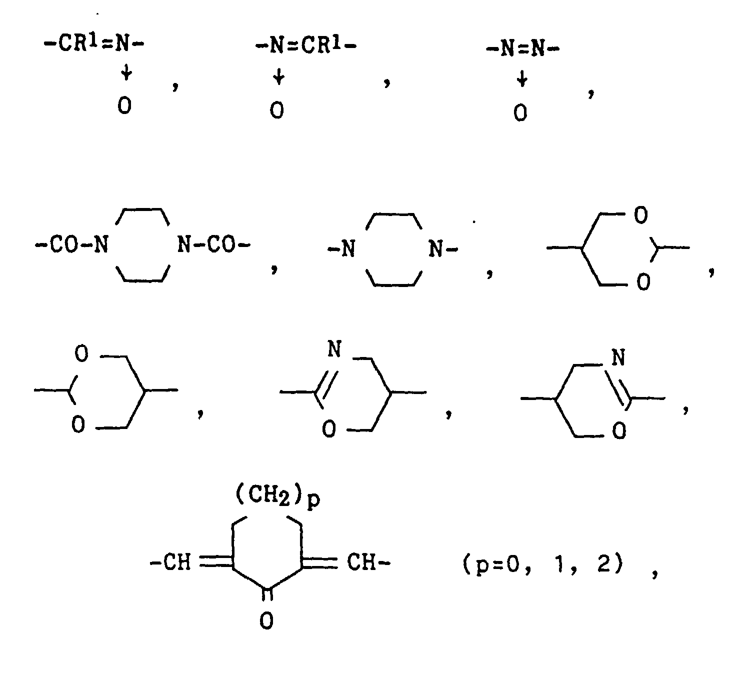 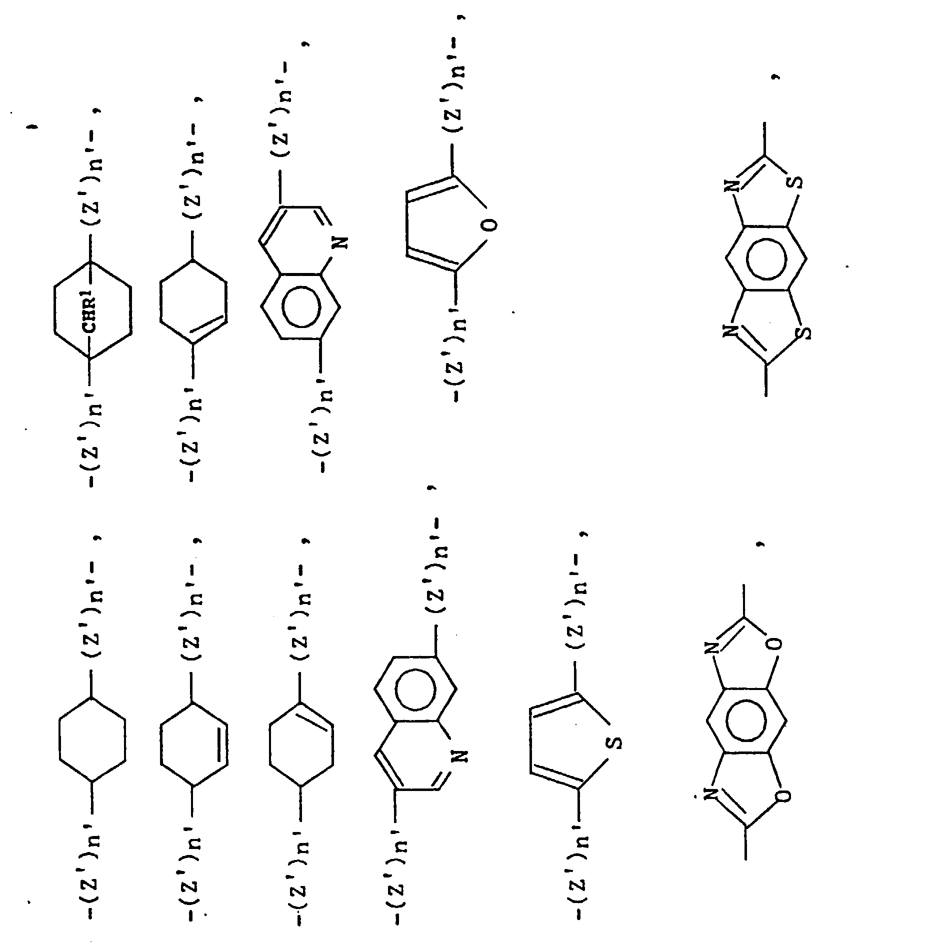 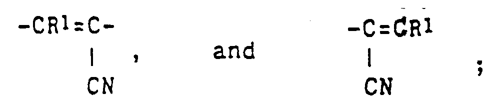 Z² is a group represented by a cyclic or bicyclic ring system containing from 5 to 12 carbon atoms and Z' and n' as previously defined; and
monoepoxide compounds containing one or more mesogenic or rodlike moieties represented by the following Formula IV: 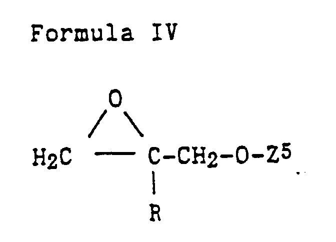 wherein Z⁵ is 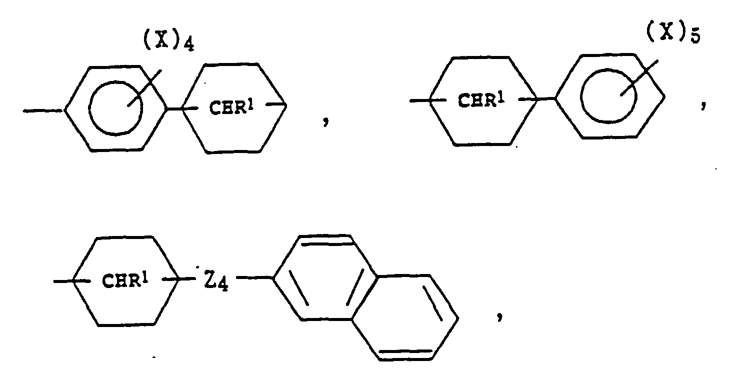 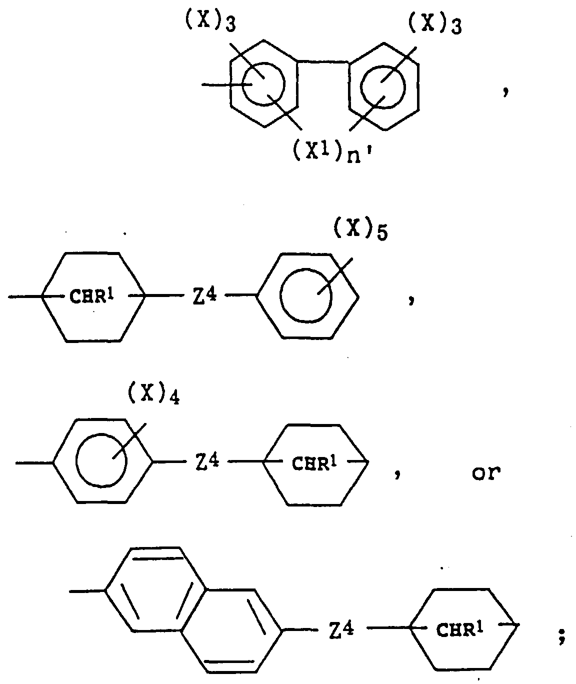 and Z⁴, R, R¹, X, n' are as defined above.
and (C) a curing amount of a suitable curing agent therefor; wherein component (A) is present in an amount suitably from 1 to 99, more suitably from 50 to 90, most suitably from 70 to 90 percent by weight based upon the combined weight of components (A) and (B) and component (B) is present in an amount suitably from 99 to 1, more suitably from 50 to 10, most suitably from 30 to 10 percent by weight based upon the combined weight of components (A) and (B).

Another aspect of the present invention pertains to curable compositions comprising (A) an advanced epoxy resin resulting from reacting (1) at least one of the epoxy resins containing one or more mesogenic or rodlike moieties represented by the above Formulas I or II with (2) at least one compound containing an average of more than one active hydrogen atom per molecule; and (B) a curing amount of a suitable curing agent therefor; wherein components (A-1) and (A-2) are present in quantities which provide a ratio of active hydrogen atoms to epoxide groups suitably from 0.01:1 to 0.95:1, more suitably from 0.05:1 to 0.80:1, most suitably from 0.10:1 to 0.5:1.

Another aspect of the present invention pertains to curable compositions comprising a blend of (A) at least one of the epoxy resins or compounds containing one or more mesogenic or rodlike moieties represented by the above Formulas I or II, (B) at least one of the polyepoxide resins represented by the above Formulas V, VI, VII, VIII, IX, X or XI, and (C) a curing amount of a suitable curing agent therefor; wherein component (A) is present in an amount suitably from 1 to 99, more suitably from 10 to 80, most suitably from 10 to 50 percent by weight based upon the combined weight of components (A) and (B) and component (B) is present in an amount suitably from 99 to 1, more suitably from 90 to 20, most suitably from 90 to 50 percent by weight based upon the combined weight of components (A) and (B).

A further aspect of the present invention pertains to products resulting from curing the aforementioned curable compositions.

A still further aspect of the present invention pertains to products resulting from the application of an electric field, a magnetic field or a shear stress during curing or processing of the aforementioned compositions.

The epoxide compositions of the present invention can be prepared by reacting the corresponding hydroxyl-containing compound with an epihalohydrin by any suitable means known to those skilled in the art. Suitable such methods are disclosed by Lee and Neville in Handbook of Epoxy Resins, McGraw-Hill, (1967).

Generally, the hydroxyl-containing compound is reacted with an epihalohydrin in the presence of a suitable catalyst and in the presence or absence of a suitable solvent at a temperature suitably from 0°C to 100°C, more suitably from 20°C to 80°C, most suitably from 20°C to 65°C; at pressures suitably from 30 mm Hg vacuum to 100 psia., more suitably from 30 mm Hg vacuum to 50 psia., most suitably from atmospheric pressure to 20 psia.; and for a time sufficient to complete the reaction, usually from 1 to 12, more usually from 1 to 5, most usually from 1 to 3 hours. This initial reaction, unless the catalyst is an alkali metal or alkaline earth metal hydroxide employed in stoichiometric quantities, produces a halohydrin intermediate which is then reacted with a basic acting compound to convert the vicinal chlorohydrin groups to epoxide groups. The resultant product is a glycidyl ether compound.

Suitable epihalohydrins which can be employed to prepare the epoxide compounds of the present invention include, for example, epihalohydrins represented by the following Formula XII: 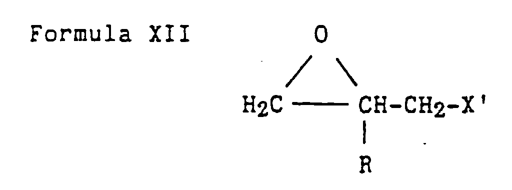 wherein R is as previously defined; and X' is a halogen. Particularly suitable such epihalohydrins include, for example, epichlorohydrin, epibromohydrin, epiiodohydrin, methylepichlorohydrin, methylepibromohydrin, methylepiiodohydrin and combinations thereof.

Suitable hydroxyl-containing compounds which can be employed to prepare the epoxide compounds of the present invention include, for example, hydroxyl-containing compounds represented by the following Formulas XIII, XIV or XV : 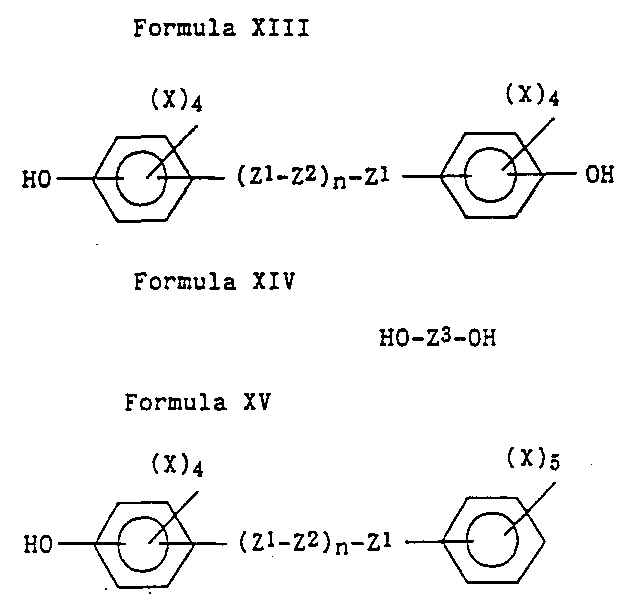 wherein at least about 80 percent of the -(Z¹-Z²)ₙ-Z¹-linkages and the hydroxyl groups are in the para position with respect to each other; n, Z¹, Z², Z³, and X are as previously defined.

Particularly suitable hydroxyl-containing compounds include, for example, 4,4'-dihydroxy-α-methylstilbene, 4,4'-dihydroxybenzanilide, 4,4'-dihydroxy-2,2'-dimethylazoxybenzene, 4,4'-dihydroxystilbene, 4,4'-dihydroxyazoxybenzene, 4,4'-dihydroxy-α-cyanostilbene, 4,4'-dihydroxydiphenylacetylene, bis(N,N'-4-hydroxyphenyl)terephthalamide, 4,4'-dihydroxy-3,3',5,5'-tetramethylstilbene, 4,4'-dihydroxy-3,3',5,5'-tetrabromostilbene, 4,4'-dihydroxy-3,3',5,5'-tetramethyl-α-methylstilbene, N-biphenyl-4-hydroxybenzamide, N-2-naphthyl-4-hydroxybenzamide, N-phenyl-4-hydroxybenzamide, N-(4'-hydroxyphenyl)benzamide, 4-hydroxystilbene, 4-hydroxy-α-methylstilbene, 4-hydroxyazobenzene, 4-hydroxy-α-cyanostilbene, 4-hydroxyazoxybenzene and combinations thereof.

Suitable catalysts which can be employed to prepare the epoxide compounds of the present invention include, for example, ammonium halides such as benzyltrimethylammonium chloride, benzyltrimethylammonium bromide, tetrabutylammonium chloride, tetrabutylammonium bromide, tetraoctylammonium chloride, tetraoctylammonium bromide, tetramethylammonium chloride, tetramethylammonium bromide and combinations thereof.

Suitable basic acting compounds which can be employed to prepare the epoxide compounds of the present invention include, for example, alkali metal or alkaline earth metal hydroxides, carbonates and bicarbonates. Particularly suitable such basic acting compounds include, for example, sodium hydroxide, potassium hydroxide, lithium hydroxide, calcium hydroxide, barium hydroxide, magnesium hydroxide, manganese hydroxide, sodium carbonate, potassium carbonate, lithium carbonate, calcium carbonate, barium carbonate, magnesium carbonate, manganese carbonate, sodium bicarbonate, potassium bicarbonate, lithium bicarbonate, calcium bicarbonate, barium bicarbonate, magnesium bicarbonate, manganese bicarbonate and mixtures thereof. Most preferred is sodium hydroxide or potassium hydroxide.

Suitable solvents which can be employed herein include, for example, alcohols, aliphatic hydrocarbons, aromatic hydrocarbons, glycol ethers, amides, sulfoxides, sulfones and combinations thereof. Particularly suitable solvents which can be employed herein include, for example, methanol, ethanol, isopropanol, hexane, heptane, octane, nonane, decane, toluene, xylene, ethylene glycol methyl ether, ethylene glycol ethyl ether, ethylene glycol n-butyl ether, ethylene glycol phenyl ether, propylene glycol methyl ether, propylene glycol phenyl ether, tripropylene glycol methyl ether, diethylene glycol methyl ether, diethylene glycol ethyl ether, diethylene glycol n-butyl ether, diethylene glycol phenyl ether, butylene glycol methyl ether, N,N-dimethylformamide, N-methylpyrrolidinone, N,N-dimethylacetamide, dimethylsulfoxide, sulfolane and combinations thereof.

The solvent is usually employed in amounts suitably from 5 to 95, more suitably from 20 to 60, most suitably from 30 to 40 percent by weight based upon the combined weight of solvent and epihalohydrin.

Suitable compounds having an average of more than one active hydrogen atom per molecule which can be employed to prepare the advanced resin compositions of the present invention include, for example, bisphenols, thiobisphenols, dicarboxylic acids and compounds containing one primary amine or amide group or two secondary amine groups such as those represented by the following Formulas XVII or XVIII: 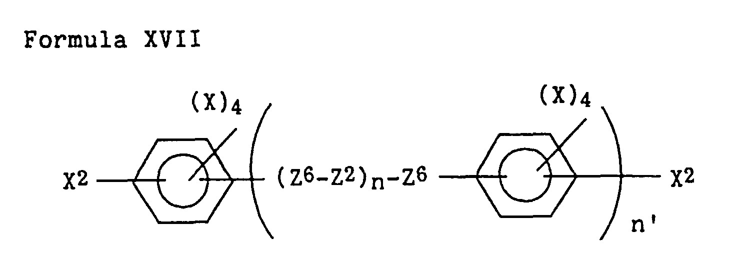 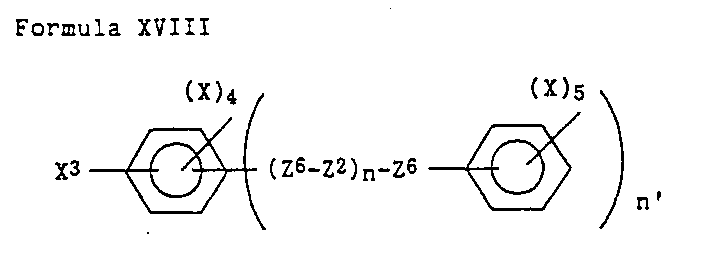 wherein X² is independently a hydroxyl, carboxylic acid, -SH, or -NHR² group; R² is an alkyl group having from 1 to 4 carbon atoms; X³ is NH₂, NH₂-SO₂-, NH₂-CO-, or NH₂-Z⁷-O-; Z⁷ is an alkyl or cycloalkyl group having from 1 to 12 carbon atoms; Z⁶ can independently be a divalent hydrocarbyl group having from 1 to 10, preferably from 1 to 4 carbon atoms, -O-, -CO-, -SO-, -SO₂-, -S-, -S-S-, -CR¹=CR¹-, -CR¹=CR¹-CR¹=CR¹-, CR¹=N-N=CR¹-, -CR¹=CR¹-CO-O-CH₂, -CR¹=CR¹-CO-O-CH₂-CH₂-, -CH₂-O-CO-CR¹=CR¹-, -CH₂-CH₂-O-CO-CR¹=CR¹-, -CR¹=CR¹-CO-O-, -O-CO-CR¹=CR¹-, -N=N-, -CO-NH-, -NH-CO-, -CO-NH-NH-CO-, -C≡C-, -C≡C-C≡C-, -CO-S-, -S-CO-, -CR¹=N-, -N=CR¹-, -CO-O-, -O-CO-, -CR¹=CR¹-CO-, -CO-CR¹=CR¹-, a direct single bond, 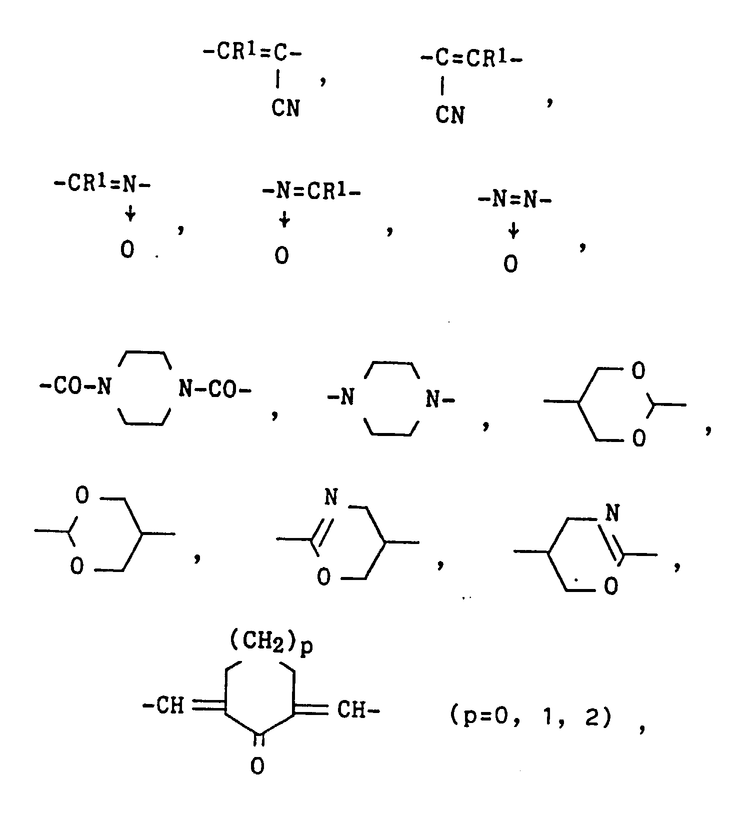 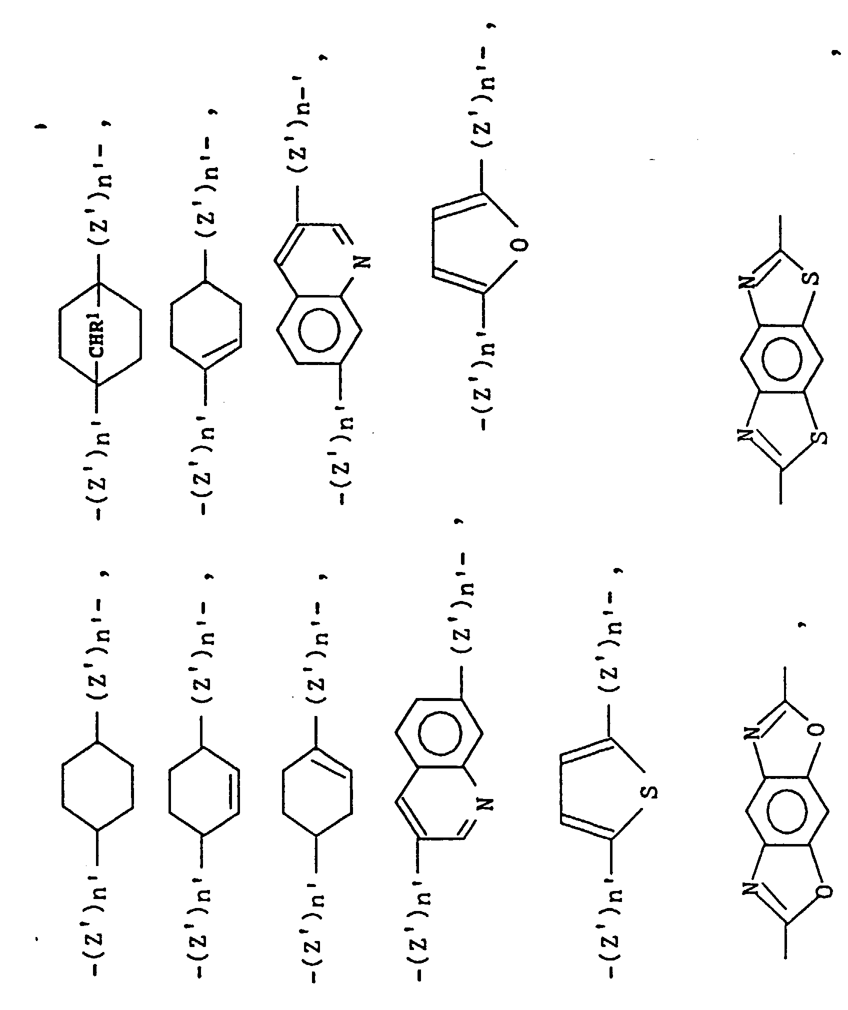 and wherein X, Z', R¹, Z², n and n' are as hereinbefore defined.

The advancement of the epoxy resins containing one or more mesogenic or rodlike moieties with compounds having an average of more than one active hydrogen per molecule is employed to linearly chain extend the resin. This linear chain extension is required for some mesogenic-containing resin compositions in order to obtain liquid crystal character. The advancement of the mesogenic or rodlike epoxy resins can also be used to increase the temperature range in which a particular resin is liquid crystalline and to control the degree of crosslinking during the final curing stage.

The epoxy resin containing one or more mesogenic or rodlike moieties and the compound having an average of more than one active hydrogen atom per molecule are reacted in amounts which provide suitably from 0.01:1 to 0.95:1, more suitably from 0.05:1 to 0.9:1, most suitably from 0.10:1 to 0.50:1 active hydrogen atoms per epoxy group.

Particularly suitable compounds having an average of more than one active hydrogen atom per molecule which can be employed herein include, for example, hydroxyl-containing compounds, carboxylic acid-containing compounds and primary amine-containing compounds. These compounds include, for example, those represented by the above-mentioned Formulas XVII and XVIII.

Particularly suitable hydroxyl-containing compounds include, for example, hydroquinone, bisphenol A, 4,4'-dihydroxydiphenylmethane, 4,4'-thiodiphenol, 4,4'-sulfonyldiphenol, 4,4'-dihydroxydiphenyl oxide, 4,4'-dihydroxybenzophenone, 1,1-bis(4-hydroxyphenyl)-1-phenylethane, 3,3',5,5'-tetrachlorobisphenol A, 3,3'-dimethoxybisphenol A, 4,4'-dihydroxybiphenyl, 4,4'-dihydroxy-α,α'-diethylstilbene, 4,4'-dihydroxy-α-methylstilbene, 4,4'-dihydroxybenzanilide, 4,4'-dihydroxy-2,2'-dimethylazoxybenzene, 4,4'-dihydroxy-α-cyanostilbene, bis(4-hydroxyphenyl)terephthalate, bis(N,N'-4-hydroxyphenyl)terephthalamide, bis(4'-hydroxybiphenyl)-terephthalate, 4,4'-dihydroxyphenylbenzoate, bis(4'-hydroxyphenyl)-1,4-benzenediimine, 4,4''-dihydroxybiphenylbenzoate, 1,4-bis(4'-hydroxyphenyl-1'-carboxamide)benzene, 1,4-bis(4'-hydroxyphenyl-1'-carboxy)benzene, 4,4'-bis(4''-hydroxyphenyl-1''-carboxy)biphenyl and mixtures thereof.

Particularly suitable carboxylic acid-containing compounds which can be employed herein include, for example, terephthalic acid, 4,4'-benzanilide dicarboxylic acid, 4,4'-phenylbenzoate dicarboxylic acid, 4,4'-stilbene-dicarboxylic acid and mixtures thereof.

Particularly suitable primary amine-containing compounds which can be employed herein include, for example, aniline, 4'-sulfon-amido-N-phenyl benzamide, 4'-sulfonamido-N'-phenyl-4-chlorobenzamide, 4-amino-1-phenylbenzoate, 4-amino-N-phenylbenzamide, N-phenyl-4-amino-phenyl-1-carboxamide, phenyl-4-aminobenzoate, biphenyl-4-aminobenzoate, 1-phenyl-4'-aminophenyl-terephthalate and mixtures thereof.

The advancement reaction can be conducted in the presence of a suitable advancement catalyst such as phosphines, quaternary ammonium compounds, phosphonium compounds and tertiary amines. Particularly suitable catalysts which can be employed herein include, for example, ethyltriphenylphosphonium chloride, ethyltriphenylphosphonium bromide, ethyltriphenylphosphonium iodide, ethyltriphenylphosphonium diacetate (ethyltriphenylphosphonium acetate·acetic acid complex), ethyltriphenylphosphonium phosphate, tetrabutylphosphonium chloride, tetrabutylphosphonium bromide, tetrabutylphosphonium iodide, tetrabutylphosphonium diacetate (tetrabutylphosphonium acetate·acetic acid complex), butyltriphenylphosphonium tetrabromobisphenate, butyltriphenylphosphonium bisphenate, butyltriphenylphosphonium bicarbonate, benzyltrimethylammonium chloride, tetramethylammonium hydroxide, triethylamine, tripropylamine, tributylamine, 2-methylimidazole, benzyldimethylamine and mixtures thereof. Many of the above-mentioned advancement catalysts are described in U. S. Patent Nos. 3,306,872; 3,341,580; 3,379,684; 3,477,990; 3,547,881; 3,637,590; 3,843,605; 3,948,855; 3,956,237; 4,048,141; 4,093,650; 4,131,633; 4,132,706; 4,171,420; 4,177,216 and 4,366,295.

The amount of advancement catalyst which can be employed herein depends, of course, upon the particular reactants and catalyst employed; however, the catalyst is usually employed in quantities of from 0.03 to 3, preferably from 0.03 to 1.5, most preferably from 0.05 to 1.5 percent by weight based upon the weight of the epoxy-containing compound.

The advancement reaction can be conducted at atmospheric, superatmospheric or subatmospheric pressures at temperatures of from 20°C to 260°C, preferably from 80°C to 240°C, more preferably from 100°C to 200°C. The time required to complete the advancement reaction depends upon the temperature employed. Higher temperatures require shorter periods of time whereas lower temperatures require longer periods of time. Generally, however, times of from 5 minutes to 24 hours, preferably from 30 minutes to 8 hours, more preferably from 30 minutes to 3 hours are suitable.

If desired, the advancement reaction can be conducted in the presence of one or more solvents. Suitable such solvents include, for example, glycol ethers, aliphatic and aromatic hydrocarbons, aliphatic ethers, cyclic ethers, ketones, esters, amides and combinations thereof. Particularly suitable solvents which can be employed herein include, for example, toluene, benzene, xylene, methyl ethyl ketone, methyl isobutyl ketone, diethylene glycol methyl ether, dipropylene glycol methyl ether, dimethylformamide, dimethyl-sulfoxide, N-methylpyrrolidinone, tetrahydrofuran, propylene glycol methyl ether and combinations thereof. The solvents can be employed in amounts of from zero to 80 percent, preferably from 20 percent to 60 percent, more preferably from 30 percent to 50 percent by weight based upon the weight of the reaction mixture.

When the epoxy resin containing one or more mesogenic or rodlike moieties and the compound having an average of more than one active hydrogen atom per molecule are reacted in amounts which provide from 0.96:1 to 1.05:1 active hydrogens per epoxy group, a relatively high molecular weight, substantially thermoplastic, resinous product is provided. Said resin composition contains little, if any, curable residual epoxide functionality, and may thus be processed using the typical processing methods employed with conventional thermoplastics, such as, for example, injection molding or extrusion. Thermosetting may, however, be induced, for example, via reaction of all or a part of the backbone secondary hydroxyl groups produced in the aforesaid advancement reaction, with a curing agent therefor. One class of said curing agents includes the di/polyisocyanates as well as the blocked di/polyisocyanates which can be induced to react with said secondary hydroxyl groups providing urethane linked crosslinks between the resin chains. An example of a specific diisocyanate especially useful herein is 4,4'-diisocyanatodiphenylmethane. When the compound having an average of more than one active hydrogen atom per molecule used in the advancement reaction is a diphenol, the resultant resinous product is a phenoxy resin.

The compositions of the present invention containing an average of more than one vicinal epoxy group per molecule can be cured with any suitable curing agent for curing epoxy resins such as primary and secondary polyamines, carboxylic acids and anhydrides thereof, aromatic hydroxyl-containing compounds, imidazoles, guanidines, urea-aldehyde resins, melamine-aldehyde resins, alkoxylated urea-aldehyde resins, alkoxylated melamine-aldehyde resins, aliphatic amines, cycloaliphatic amines, aromatic amines which can be employed herein and combinations thereof. Particularly suitable curing agents include, for example, methylene dianiline, dicyandiamide, ethylene diamine, diethylenetriamine, triethylenetetramine, tetraethylenepentamine, urea-formaldehyde resins, melamine-formaldehyde resins, methylolated urea-formaldehyde resins, methylolated melamine-formaldehyde resins, phenol-formaldehyde novolac resins, cresol-formaldehyde novolac resins, sulfanilamide, diaminodiphenylsulfone, diethyltoluenediamine, t-butyltoluenediamine, bis-4-aminocyclohexylmethane, isophoronediamine, diaminocyclohexane, hexamethylenediamine, piperazine, aminoethylpiperazine, 2,5-dimethyl-2,5-hexanediamine, 1,12-dodecanediamine, tris-3-aminopropylamine and combinations thereof.

The curing agents are employed in amounts which will effectively cure the composition; however, these amounts will depend upon the particular epoxy resin and curing agent employed. Generally, suitable amounts of curing agent which can be employed herein include, for example, from 0.95:1 to 1.2:1 equivalents of curing agent per equivalent of epoxy resin.

The mesogenic or rodlike diepoxides of the present invention can also be employed for the purpose of improving the properties of polyepoxide resins not containing mesogenic or rodlike moieties. Generally, suitable amounts of mesogenic or rodlike epoxy resins are from 1 to 99, more suitably from 10 to 80, most suitably from 10 to 50 weight percent based on the total weight of the combined resins. Representative of the other polyepoxide resins include, for example, the diglycidyl ethers of resorcinol, bisphenol A, 4,4'-dihydroxydiphenylmethane, 3,3',5,5'-tetrabromobisphenol A, 4,4'-thiodiphenol, 4,4'-sulfonyldiphenol, 4,4'-dihydroxydiphenyl oxide, 1,1-bis(4-hydroxyphenyl)-1-phenylethane, 3,3',5,5'-tetrachlorobisphenol A, 3,3'-dimethoxybisphenol A; the triglycidyl ether of tris(hydroxyphenyl)methane; the polyglycidyl ether of a phenol or substituted phenol-aldehyde condensation product (novolac); the polyglycidyl ether of a dicyclopentadiene or an oligomer thereof and phenol condensation product; the advancement reaction products of the aforesaid di- and polyglycidyl ethers with aromatic di- or polyhydroxyl- or carboxylic acid-containing compounds including, for example, bisphenol A (4,4'-isopropylidenediphenol), o-, m-, p-dihydroxybenzene, 2,4-dimethylresorcinol, 4-chlororesorcinol, tetramethylhydroquinone, 1,1-bis(4-hydroxyphenyl)ethane, 1,1-bis(4-hydroxyphenyl)-1-phenylethane, 4,4'-dihydroxydiphenyl ether, 3,3',5,5'-tetramethyldihydroxydiphenyl ether, 3,3',5,5'-dichlorodihydroxydiphenyl ether, 4,4'-bis-(p-hydroxyphenyl isopropyl)diphenyl ether, 4,4'-bis-(p-hydroxyphenoxy)benzene, 4,4'-bis(p-hydroxyphenoxy)diphenyl ether, 4,4'-bis(4-(4-hydroxyphenoxy)-phenyl sulfone)diphenyl ether, 4,4'-dihydroxydiphenyl sulfone, 4,4'-dihydroxydiphenyl sulfide, 4,4'-dihydroxydiphenyl disulfide, 2,2'-dihydroxydiphenyl sulfone, 4,4'-dihydroxydiphenyl methane, 1,1-bis(p-hydroxyphenyl)cyclohexane, 4,4'-dihydroxybenzophenone, phloroglucinol, pyrogallol, 2,2',5,5'-tetrahydroxydiphenyl sulfone, tris(hydroxyphenyl)methane, dicyclopentadiene diphenol, tricyclopentadiene diphenol; and mixtures thereof.

During processing and/or cure of the epoxy resin compositions into a part, electric or magnetic fields or shear stresses can be applied for the purpose of orienting the liquid crystal moieties contained or developed therein which in effect improves the mechanical properties. As specific examples of these methods, Finkelmann, et al, Macromol. Chem., 180, 803-806 (March 1979) induced orientation in thermotropic methacrylate copolymers containing mesogenic side chain groups decoupled from the main chain via flexible spacers in an electric field. Orientation of mesogenic side chain groups decoupled from the polymer main chain via flexible spacers in a magnetic field has been demonstrated by Roth and Kruecke, Macromol. Chem., 187, 2655-2662 (November 1986). Magnetic field induced orientation of mesogenic main chain containing polymers has been demonstrated by Moore, et al, ACS Polymeric Material Sciences and Engineering, 52, 84-86 (April-May 1985). Magnetic and electric field orientation of low molecular weight mesogenic compounds is discussed by W. R. Krigbaum in Polymer Liquid Crystals, pages 275-309 (1982) published by Academic Press, Inc.

In addition to orientation by electric or magnetic fields, polymeric mesophases can be oriented by shear forces which are induced by flow through dies, orefices, and mold gates. A general discussion for orientation of thermotropic liquid crystal polymers by this method is given by S. K. Garg and S. Kenig in High Modulus Polymers, pages 71-103 (1988) published by Marcel Dekker, Inc. For the mesomorphic systems based on the epoxy resin compositions, this shear orientation can be produced by processing methods such as injection molding, extrusion, pultrusion, filament winding, filming and prepreging.

The mesogenic or rodlike epoxy resins of the present invention can be blended with other materials such as solvents or diluents, fillers, pigments, dyes, flow modifiers, thickeners, reinforcing agents, mold release agents, wetting agents, stabilizers, fire retardant agents, surfactants and combinations thereof.

These additives are added in functionally equivalent amounts, e.g., the pigments and/or dyes are added in quantities which will provide the composition with the desired color; however, these pigments and/or dyes are suitably employed in amounts of from zero to 20, more suitably from 0.5 to about 5, most suitably from 0.5 to 3 percent by weight based upon the weight of the total blended composition.

Solvents or diluents which can be employed herein include, for example, hydrocarbons, ketones, glycol ethers, aliphatic ethers, cyclic ethers, esters, amides and combinations thereof. Particularly suitable solvents or diluents which can be employed herein include, for example, toluene, benzene, xylene, methyl ethyl ketone, methyl isobutyl ketone, diethylene glycol methyl ether, dipropylene glycol methyl ether, dimethylformamide, N-methylpyrrolidinone, tetrahydrofuran, propylene glycol methyl ether and combinations thereof.

The modifiers such as thickeners and flow modifiers can be suitably employed in amounts of from zero to 10, more suitably from 0.5 to 6, most suitably from 0.5 to 4 percent by weight based upon the weight of the total composition.

Reinforcing materials which can be employed herein include natural and synthetic fibers in the form of woven fabric, mats, monofilament, multifilament, unidirectional fibers, rovings, random fibers or filaments, inorganic fillers or whiskers and hollow spheres. Suitable reinforcing materials which can be employed herein include, for example, glass, ceramics, nylon, rayon, cotton, aramid, graphite, polyalkylene terephthalates, polyethylene, polypropylene, polyesters and combinations thereof.

Suitable fillers which can be employed herein include, for example, inorganic oxides, ceramic microspheres, plastic microspheres, glass microspheres, inorganic whiskers, CaCO₃ and combinations thereof.

The fillers can be employed herein in amounts suitably from zero to 95, more suitably from 10 to 80, most suitably from 40 to 60 percent by weight based upon the weight of the total composition.

The following examples are illustrative of the present invention.

### Comparative Experiment A

### 1. Preparation of a Neat Resin Casting of the Diglycidyl Ether of Bisphenol A

A diglycidyl ether of bisphenol A (50.00 grams) having an epoxide equivalent weight of 175.6 was combined with an equivalent amount of a curing agent, sulfanilamide (12.26 grams). This blend was heated in a 170°C convection oven for 20 minutes to dissolve all of the sulfanilamide. This resinous mixture was then transferred to a 100°C oven where it was allowed to cool for 20 minutes. A 70 weight percent solution of tetrabutylphosphonium acetate·acetic acid complex in methanol (0.28 grams, 4480 ppm based on the total weight of the resin mixture) was then added to promote the eventual cure. The resin was next degassed in a vacuum bell jar and then poured into an aluminum mold (dimensions =(4.5 inch x 5.0 inch x 0.125 inch) 114.3 mm x 127 mm x 3.18 mm) which was contained in a 120°C convection oven. After one hour at 120°C, the temperature of the oven was increased and held for one hour at 150°C and then held for one hour at 180°C. After one hour at 180°C, the temperature of the oven was increased to 200°C where it was held for 1.5 hours before cooling to room temperature. At room temperature a translucent, neat resin casting was obtained from the mold. Differential scanning calorimetry analysis for this polymer showed complete cure. The glass transition temperature, as determined by differential scanning calorimetry, was 185°C. The glass transition temperature, which was also determined by dynamic mechanical analysis, was 186°C and was in close agreement with the value obtained by differential scanning calorimetry. The tensile storage modulus at 40°C which was obtained from the dynamic mechanical analysis was 670 MPa (97,150 psi). The flexural strength and modulus determined for this polymer by ASTM Method D790 were 137,7 kPa (19,960 psi) and 3257 MPa (472 ksi), respectively.

### 2. Preparation of Cast Films of the Diglycidyl Ether of Bisphenol A

A diglycidyl ether of bisphenol A (30.00 grams) having an epoxide equivalent weight of 179.5 was combined with an equivalent amount of a curing agent, sulfanilamide (7.20 grams). This blend was heated in a 170°C convection oven for 20 minutes to dissolve all of the sulfanilamide. This resinous mixture was then transferred to a 100°C oven where it was allowed to cool for 20 minutes. A 70 weight percent solution of tetrabutylphosphonium acetate·acetic acid complex in methanol (0.18 grams, 4810 ppm based on the total weight of the resin mixture) was then added to promote the eventual cure. This resin was next degassed in a vacuum bell jar and then poured into a stainless steel mold (dimensions = (7.5 inch x 0.5 inch x 0.021 inch); 190.5 mm x 12.7 mm x 0.53 mm) located in a 120°C convection oven. The mold was then transferred to a mechanical press heated to 120°C. Pressure (approximately 2000 psi) was applied to this mold within 5 minutes. After one hour at 120°C, the temperature of the press was increased and held for one hour at 150° and then held for one hour at 180°C. After one hour at 180°C, the temperature of the press was increased to 200°C where it was held for 1.5 hours before cooling to room temperature. After cooling to room temperature, a translucent film was obtained from the mold. The glass transition temperature for this polymer was 180°C as determined by thermal mechanical analysis. The average tensile strength and modulus for 5 castings prepared using this procedure were 0.74 MPa (8803 psi) (standard deviation = 5.9 MPa (860 psi) and 3553.5 MPa (515 ksi) (standard deviation = 62 MPa (9.0 ksi)), respectively.

### Comparative Experiment A

### 3. Preparation of a Neat Resin Casting of the Diglycidyl Ether of Bisphenol A and Measurement of Chemical Resistance

A diglycidyl ether of bisphenol A (50.00 grams) having an epoxide equivalent weight of 175.6 was combined with an equivalent amount of a curing agent, sulfanilamide (12.26 grams). This blend was heated in a 170°C convection oven for 20 minutes to dissolve all of the sulfanilamide. This resinous mixture was then transferred to a 100°C oven where it was allowed to cool for 30 minutes. A 70 weight percent solution of tetrabutylphosphonium acetate·acetic acid complex in methanol (0.28 grams, 4480 ppm based on the total weight of the resin mixture) was then added to promote the eventual cure. The resin was next degassed in a vacuum bell jar and then poured into an aluminum mold (dimensions = (4.5 inches x 5.0 inches x 0.125 inches) 114.3 mm x 127 mm x 3.18 mm) which was preheated in a 120°C convection oven. After 1.5 hours at 120°C, the temperature of the oven was increased and held for one hour at 150°C and then held for one hour at 180°C. After one hour at 180°C, the temperature of the oven was increased to 200°C where it was held for 2 hours before cooling to room temperature. At room temperature, a translucent neat resin casting was obtained from the mold. Differential scanning calorimetry analysis for this polymer showed complete cure. The glass transition temperature, as determined by differential scanning calorimetry was 188°C. From the casting obtained, test coupons which were approximately 1.27 cm (0.5 inches) wide by 3.81 cm (1.5 inches) long were cut and individually weighed. These coupons were separately immersed in methylene chloride, methyl ethyl ketone, dimethylformamide and bleach, then maintained therein at room temperature (approximately 23°C). After immersion for a measured time, the coupons were removed from the chemical, wiped dry and then weighed. For these neat resin coupons of the diglycidyl ether of bisphenol A cured with sulfanilamide, the percent weight gains were 26.6 after 30 days in methylene chloride, 4.1 after 60 days in methyl ethyl ketone, 5.7 after 30 days in dimethylformamide and 1.0 after 30 days in bleach. The results are reported in Table IV.

### Comparative Experiment A

### 4. Preparation of an Injection Molded, Graphite Composite of the Diglycidyl Ether of Bisphenol A

A diglycidyl ether of bisphenol A (4.95 grams) having an epoxide equivalent weight of 179.5 was placed in a 160°C oven. After the resin had been in the oven for 8 minutes, an equivalent amount of a curing agent, sulfanilamide (1.187 grams), was added. This mixture was periodically stirred over the next 10 minutes to dissolve all the sulfanilamide into the resin. Chopped graphite fibers (1.083 grams; 6.3 mm (1/4 inch) long were then added. The graphite fibers used were obtained from Fortafil Fibers, Inc. and the manufacturer's designation for this product was Fortafil™ 3 (c) 1/4 07. After adding the graphite fibers (15 weight percent based on total components), they were mixed with the resin over the next 5 minutes to produce a blend. This blend was next degassed using a vacuum bell jar and then transferred to the reservoir of an injection molder which was temperature controlled at 120°C. After 20 minutes, heating of the molder reservoir was discontinued. When the molder reservoir had cooled to 100°C, the mixture was injected through a (1/8 inch x 3/32 inch) 3.0 mm x 2.3 mm rectangular orifice into a mold (dimensions = (3 inches x 0.5 inches x 0.125 inches); 76.2 mm x 12.7 mm x 3.18 mm which was preheated to 85°C. This mold was then immediately transferred to an 85°C oven. After 14.5 hours at 85°C, the oven temperature was increased to 230°C over a 7 hour period. The oven temperature was then maintained at 230°C for 4 hours before cooling to room temperature. At room temperature a graphite composite of the diglycidyl ether of bisphenol A cured with sulfanilamide was obtained from the mold. The flexural strength and modulus for this composite were 148 MPa (21,450 psi) and 8135 MPa (1,179 ksi), respectively. Differential scanning calorimetry was performed at a heating rate of 10°C per minute for a sample of the composite (approximately 20 milligrams). This analysis showed a glass transition temperature of 190°C. The results are reported in Table V.

### Example 1

### A. Synthesis of 4,4'-Dihydroxy-alphamethylstilbene

Phenol (188.22 grams, 2.0 moles) and chloroacetone (102.81 grams, 1.0 mole as chloroacetone) were added to a reactor and cooled to -10°C with stirring. The chloroacetone used was a technical grade containing 90 percent chloroacetone, 2.5 percent acetone, 6.5 percent 1,1-dichloroacetone and 1.0 percent 1,3-dichloroacetone. Concentrated sulfuric acid (98.08 grams, 1.0 mole) was added dropwise to the stirred solution over a one hour period in order to maintain the reaction temperature between -10°C and -11°C. After two hours of post reaction at the -10°C temperature, the resultant viscous orange oil product was mixed with 500 milliliters of iced deionized water. The oil product was separated then washed with a second 500 milliliter portion of chilled deionized water. After separation, the recovered oil product was added to a 2-liter beaker along with 250 milliliters of ethanol and stirred to provide a solution. Deionized water (250 milliliters) was added to the stirred solution and heating commenced. As the temperature of the mixture increased, the stirred mixture began to clear. Each time clearing was observed, sufficient deionized water was added to induce cloudiness, followed by continuation of the mixing and heating. Once the temperature reached 90°C, a massive precipitation of white crystalline plates occurred. At this time, heating and stirring ceased and the mixture was chilled to 5°C and held therein for 12 hours. The crystalline product was recovered by filtration, washed with two 150 milliliter portions of deionized water, then dried at 90°C and 5 mm Hg to a constant weight of 103 grams. Nuclear magnetic resonance spectroscopy and infrared spectrophotometric analysis confirmed the product structure for 4,4'-dihydroxy-α-methylstilbene.

### B. Epoxidation of 4,4'-Dihydroxy-α-methylstilbene

4,4'-dihydroxy-α-methylstilbene (113.13 grams, 1.0 hydroxyl equivalent) prepared using the method of A above, epichlorohydrin (462.65 grams, 5 moles), deionized water (40.23 grams, 8.0 percent by weight of the epichlorohydrin used) and isopropanol (249.12 grams, 35 percent by weight of the epichlorohydrin used) were added to a reactor and heated to 55°C with stirring under a nitrogen atmosphere. Once the 55°C reaction temperature was achieved, sodium hydroxide (36.0 grams, 0.90 mole) dissolved in deionized water (144 grams) was added dropwise to the reactor over a 40 minute period in order to maintain a reaction temperature between 55 and 59°C. Ten minutes after completion of the aqueous sodium hydroxide addition, the stirring was stopped and the aqueous layer which separates from the reaction mixture was pipetted off and discarded. Stirring was resumed and after a total of twenty minutes following completion of the initial aqueous sodium hydroxide addition, a second solution of sodium hydroxide (16.0 grams, 0.40 mole) dissolved in deionized water (64 grams) was added to the reactor over a twenty minute period so as to maintain the 55°C reaction temperature. Fifteen minutes after completion of the aqueous sodium hydroxide addition, the recovered reaction mixture was added to a separatory funnel and washed with 750 milliliters of deionized water. The separated organic layer was washed a second time (750 milliliters deionized water), recovered and then rotary evaporated under vacuum for 45 minutes at 110°C then 30 minutes at 130°C. The product was recovered (166.5 grams) as a crystalline off-white solid with an epoxide equivalent weight of 181.46.

### C. Characterization of Liquid Crystallinity in the Diglycidyl Ether of 4,4'-Dihydroxy-α-methylstilbene

A portion (10.84 milligrams) of the diglycidyl ether of 4,4'-dihydroxy-alpha-methylstilbene from B above was analyzed by differential scanning calorimetry using a heating rate of 10°C per minute and a temperature range of 30°C to 150°C. The results are reported in Table I.

**TABLE I**

| DIFFERENTIAL SCANNING CALORIMETRY ANALYSIS OF THE DIGLYCIDYL ETHER OF 4,4'-DIHYDROXY-a-METHYLSTILBENE | | | |
|---|---|---|---|
| Cycle Designation | Observed Transition (°C) Temperatures midpoint/range | Enthalpy (J/g) | Comments |
| First heat (30 to 150°C) | 73/55-84 | 6.3 | --- |
| | 122/84-130 | 41.8 | --- |
| First cooling (150 to 30°C) | -/81-52 | --- | 2 unresolved broad, flat peaks |
| Second heat (30 to 150°C) | 84/48-92 (shoulder at 69) | 31.4 | --- |
| | 124/108-132 | 3.6 | --- |
| Second cooling (150 to 30°C) | -/81-52 | --- | 2 unresolved broad, flat peaks |

Analysis of the diglycidyl ether via polarized light microscopy was completed using a microscope equipped with a programmable hot stage using a heating rate of 20°C per minute. The results are reported in Table II.

**TABLE II**

| POLARIZED LIGHT MICROSCOPY ANALYSIS OF THE DIGLYCIDYL ETHER OF 4,4'-DIHYDROXY-α-METHYLSTILBENE | | |
|---|---|---|
| Cycle Designation | Observed Transition Temperatures (°C) | Comments |
| First heat | 109 | First fluidity noted. |
| | 137 | Isotropization completed. |
| First cooling | 91 | First mobile nematic droplets observed. |
| | 51 | First crystallization noted. |
| Second heat | 63 | First fluidity noted. |
| | 78 | Flows to nematic texture. |
| | 86 | Isotropization completed but minor crystalline fraction still present. |
| | 133 | All crystalline fraction melted. |
| Second cooling | 91 | First mobile nematic droplets observed. |
| | 51 | First crystallization noted. |

The diglycidyl ether is a monotropic liquid crystal with a nematic texture. Two fractions are present: the higher melting crystalline fraction becomes liquid crystalline at 91°C (microscopic observation) followed by the development of liquid crystallinity in the second, lower melting fraction. This accounts for the broadness and overlapping observed in the cooling cycles by differential scanning calorimetry.

### D. Preparation of a Neat Resin Casting of the Diglycidyl Ether of 4,4'-Dihydroxy-α-methylstilbene

A diglycidyl ether of 4,4'-dihydroxy-α-methylstilbene (36.56 grams, epoxide equivalent weight of 182.0) prepared using the same method as in B was melted in a 160°C convection oven. An equivalent amount of a curing agent, sulfanilamide (8.65 grams), was then added to the resin. After all of the sulfanilamide had dissolved (approximately 20 minutes), the mixture was then degassed in a vacuum bell jar and poured into an aluminum mold (dimensions = (4.5 inch x 5.0 inch x 0.125 inch) 114.3 mm x 127 mm x 3.18 mm) located in a 100°C oven. After two hours at 100°C, the temperature of the oven was increased and held for one hour at the following temperatures: 120°C, 140°C, 160°C, 180°C, 200°C and 220°C. Upon cooling to room temperature, an opaque, cream colored casting was obtained from the mold. Differential scanning calorimetry analysis of this polymer showed no discernable transitions or exothermic activity to 280°C. The apparent glass transition temperature for this polymer, as determined by dynamic mechanical analysis, was 260°C which was 74°C higher than that obtained for the diglycidyl ether of bisphenol A in Comparative Experiment A-1. The dynamic mechanical analysis also gave a tensile storage modulus of 950 MPa (137,750 psi) at 40°C for this polymer which was 40 percent higher than that obtained for the diglycidyl ether of bisphenol A. The flexural strength and modulus obtained for this casting using ASTM Method D790 were 130,4 MPa (18,900 psi) and 3401 MPa (493 ksi), respectively. These flexural properties were comparable to those obtained for the diglycidyl ether of bisphenol A of Comparative Experiment A-1. The results are reported in Table III.

**TABLE III**

| PROPERTIES OF NEAT RESIN CASTINGS | | |
|---|---|---|
| PROPERTY | DIGLYCIDYL ETHER OF BISPHENOL A (Comparative Experiment A-1) | DIGLYCIDYL ETHER OF 4,4'-DIHYDROXY--α-METHYL-STILBENE (Example 1-D) |
| Glass Transition Temperature, °C (Dynamic Mechanical Analysis) | 186 | 260 (apparent) |
| Tensile Storage Modulus, (Dynamic Mechanical Analysis) | 670,7 MPA (97.2) ksi | 950,8 MPa (137.8 ksi) |
| Flexural Strength, | 137,7 MPa (19,960psi) | 130,4 MPa (18,900 psi) |
| Flexural Modulus, ksi | 3257 MPa (472 ksi) | 3401 MPa (493 ksi) |

In addition to the above analysis, a sample of the resinous mixture of the diglycidyl ether of 4,4'-dihydroxy-α-methylstilbene and sulfanilamide which was used to prepare this casting was viewed under an optical microscope (70X magnification) using a cross polarized light source. This sample was heated to 100°C and held at this temperature to duplicate the initial curing conditions of the casting. After 30 minutes at 100°C, a nematic liquid crystalline texture begins to develop. At this stage of resin advancement, shear pressure was applied by moving the microscope slide cover under which the sample was contained. With the application of this shear pressure, birefringent, fibrous domains were observed to form and orient in one direction.

### Example 1

### E. Preparation of a Neat Resin Casting of the Diglycidyl Ether of 4,4'-Dihydroxy-α-Methylstilbene and Measurement of Chemical Resistance

A diglycidyl ether of 4,4'-dihydroxy-α-methylstilbene (19.28 grams, epoxide equivalent weight of 179.2) prepared using the same method as in B was melted in a 160°C convection oven. After melt of this resin had occurred, an equivalent amount of a curing agent, sulfanilamide (4.65 grams), was added. This mixture was periodically stirred over the next 10 minutes to dissolve all the sulfanilamide into the resin. The mixture was then degassed in a vacuum bell jar and poured into an aluminum mold (dimensions = (3 inch x 3 inch x 0.125 inch); 76.2 mm x 76.2 mm x 3.18 mm) preheated in a 120°C oven. After two hours at 120°C, the temperature of the oven was increased and held for one hour at the following temperatures: 150°C, 180°C and 200°C. After one hour at 200°C, the temperature of the oven was increased to 225°C where it was held for 2 hours before cooling to room temperature. Upon cooling to room temperature, an opaque, cream colored casting was obtained from the mold. Differential scanning calorimetry analysis of this polymer showed no discernable transitions or exothermic activity to 270°C. From the casting obtained, test coupons which were approximately long (12.7 mm x 38,1) (0.5 inch wide by 1.5 inch) were cut and individually weighed. These coupons were separately immersed in methylene chloride, methyl ethyl ketone, dimethylformamide and bleach, then maintained therein at room temperature (approximately 23°C). After immersion for a measured time, the coupons were removed from the chemical, wiped dry and then weighed. For these neat resin coupons of the diglycidyl ether of 4,4'-dihydroxy-α-methylstilbene cured with sulfanilamide, the percent weight gains were 0.56 after 30 days in methylene chloride, 0.02 after 60 days in methyl ethyl ketone, 0.67 after 30 days in dimethylformamide and 0.25 after 30 days in bleach. The results are reported in Table IV.

**TABLE IV**

| Solvent Absorption for Neat Resin Castings | | |
|---|---|---|
| | Diglycidyl Ether of Bisphenol A (Comparative Experiment A-3) | Diglycidyl Ether of 4,4'-Dihydroxy-α-methylstilbene (Example 1-E) |
| Percent Weight Gain After 30 Days Immersion in Methylene Chloride | 26.6 | 0.56 |
| Percent Weight Gain After 60 Days Immersion in Methyl Ethyl Ketone | 4.1 | 0.02 |
| Percent Weight Gain After 30 Days Immersion in Dimethylformamide | 5.7 | 0.67 |
| Percent Weight Gain After 30 Days Immersion in Bleach | 1.0 | 0.25 |

### Example 1

### F. Preparation of An Injection Molded, Graphite Composite of the Diglycidyl Ether of 4,4'-Dihydroxy-α-Methylstilbene

A diglycidyl ether of 4,4'-dihydroxy-α-methylstilbene (4.95 grams, epoxide equivalent weight of 177.25) prepared using the same method as in B was placed in a 160°C oven. After melt of this resin had occurred (approximately 8 minutes), an equivalent amount of a curing agent, sulfanilamide (1.202 grams), was added. This mixture was periodically stirred over the next 10 minutes to dissolve all the sulfanilamide into the resin. Chopped graphite fibers (1.086 grams; 1/4 inch long (6.3 mm)) were then added. The graphite fibers used were obtained from Fortafil Fibers, Inc. and the manufacturer's designation for this product was Fortafil 3(c) 1/4 07. After adding the graphite fibers (15 weight percent based on total components), they were mixed with the resin over the next 5 minutes to produce a blend. This blend was next degassed using a vacuum bell jar and then transferred to the reservoir of an injection molder which was temperature controlled at 120°C. After 20 minutes, heating of the molder reservoir was discontinued. When the molder reservoir had cooled to 100°C, the mixture was injected through a (1/8 inch x 3/32 inch) 3.0 mm x 2.3 mm rectangular orifice into a mold (dimensions = (3 inch x 0.5 inch x 0.125 inch); 76.2 mm x 12.7 mm x 3.18 mm) which was preheated to 85°C. This mold was then immediately transferred to an 85°C oven. After 17 hours at 85°C, the oven temperature was increased to 230°C over a 7 hour period. The oven temperature was then maintained at 230°C for 6 hours before cooling to room temperature.

At room temperature a graphite composite of the diglycidyl ether of 4,4'-dihydroxy-α-methylstilbene cured with sulfanilamide was obtained from the mold. The flexural strength and modulus for this composite were 191,4 MPa (27,740 psi) and 11861 MPa (1,719 ksi), respectively. Differential scanning calorimetry was performed at a heating rate of 10°C per minute for a sample of the composite (approximately 20 milligrams). This analysis showed no discernable glass transition temperature to 300°C. The results are reported in Table V.

**TABLE V**

| Properties for Injection Molded, Graphite Composites | | |
|---|---|---|
| | Diglycidyl Ether of Bisphenol A (Comparative Experiment A-4) | Diglycidyl Ether of 4,4'-Dihydroxy-α-Methylstilbene (Example 1-F) |
| Glass Transition Temperature, °C (Differential Scanning Calorimetry) | 190 | None Detected |
| Flexural Strength, | 148 MPa (21,450 psi) | 191,4 MPa (27,740 psi) |
| Flexural Modulus, | 8135 MPa (1,179 ksi) | 11861 MPa (1,719 ksi) |

### Example 1

### G. Preparation of a Cured Resin Composition Based on a Mixture of the Diglycidyl Ether of 4,4'-Dihydroxy-α-Methylstilbene and the Diglycidyl Ether of Bisphenol A

The diglycidyl ether of 4,4'-dihydroxy-α-methylstilbene (4.9129 grams, epoxide equivalent weight = 179.1) prepared using the same method as in B was melted in a 160°C convection oven. After the resin had melted, an equivalent amount of curing agent sulfanilamide (1.1809 grams) was added. This blend was periodically stirred over the next 15 minutes to dissolve all the sulfanilamide. After the sulfanilamide had dissolved, the mixture was removed from the oven and cooled to room temperature. At room temperature, part of this material (0.2875 grams) was added to an aluminum cup containing 0.2875 grams of a mixture consisting of a diglycidyl ether of bisphenol A (epoxide equivalent weight = 175.6) and an equivalent amount of dissolved curing agent (sulfanilamide). The aluminum cup containing the diglycidyl ether of 4,4'-dihydroxy-α-methylstilbene and the diglycidyl ether of bisphenol A was then placed in a 120°C convection oven. Another aluminum cup containing 0.46 grams of a control (a mixture of the diglycidyl ether of bisphenol A, an equivalent amount of sulfanilamide and 4480 ppm catalyst (tetrabutylphosphonium acetate·acetic acid complex, 70 weight percent in methanol), prepared using the same procedure as in Comparative Experiment A-1) was also placed in the 120°C oven at the same time. In the oven, these two resin systems were stirred during the first 10 minutes and then allowed to gel. After 3 hours at 120°C, the oven temperature was increased and held for one hour at the following temperatures: 150°C, 180°C and 200°C. After 1 hour at 200°C, the oven temperature was increased to 225°C where it was held for 2 hours before cooling to room temperature. At room temperature, the two cured resin compositions were removed from the aluminum cups and differential scanning calorimetry analysis was performed for these polymers to determine the glass transition temperatures. The glass transition temperature for the control, the diglycidyl ether of bisphenol A cured with sulfanilamide, was 185°C which reproduced the value obtained in Comparative Experiment A-1. The glass transition temperature for the mixture of the diglycidyl ether of 4,4'-dihydroxy-α-methylstilbene and the diglycidyl ether of bisphenol A cured with sulfanilamide was 211°C which represents a 26°C improvement over the control.

### Example 2

### A. Synthesis of 4,4'-Dihydroxybenzanilide

One hundred grams of 4,4'-dihydroxybenzophenone (0.467 mole) was added to 300 ml of ethanol in a stirred, 1-liter Erlenmeyer flask. After the 4,4'-dihydroxybenzophenone had dissolved, a solution consisting of 48.6 grams of hydroxylamine hydrochloride (0.699 mole) and 57.4 grams of sodium acetate (0.700 mole) in 70 ml of deionized water was added followed by an additional 100 ml of ethanol. This mixture was stirred and heated on a hot plate to a gentle refluxing condition (75°C). After heating for 4 hours, the solution was allowed to cool to room temperature with stirring and then filtered. One hundred ml of ethanol was used to wash the filter cake. The total filtrate obtained (600.4 grams) was then concentrated to a weight of 219.2 grams by evaporation of the ethanol and water. This solution was then placed in a stirred 1-liter Erlenmeyer flask to which 600 ml of deionized water was added. With the addition of the deionized water, a white precipitate was formed. After 30 minutes of stirring, this solution was filtered. The solids obtained weighed 98.22 grams after drying. Sixty-six grams of this material (4,4'-dihydroxybenzophenone oxime, 0.288 mole) was added to 330 ml of glacial acetic acid in a 500 ml round bottom flask equipped with a stirrer, water cooled condensor, nitrogen purge and heating mantle. A catalytic amount of toluenesulfonic acid (1.85 grams, 0.027 mole) was next added and the reaction mixture was then heated to 83°C. After heating for approximately 2 hours, a precipitate was formed which was stirred for an additional 2 hours at 87°C. Twenty-five ml of deionized water was next added and after 30 minutes, the contents of the reaction flask were transferred to a stirred, 1-liter Erlenmeyer flask. Immediately following this transfer, 400 ml of deionized water was added. This solution was stirred for 45 minutes and then filtered. The filter cake obtained was washed with 800 ml of deionized water and then dried. The resultant solids, which were a light beige color, weighed 54.82 grams. Fourier transform infrared analysis of this product showed absorbances which were indicative of the structure for 4,4'-dihydroxybenzanilide. Differential scanning calorimetry analysis showed a sharp melt endotherm at 273°C for the 4,4'-dihydroxybenzanilide thus obtained.

### B. Epoxidation of 4,4'-Dihydroxybenzanilide

4,4'-dihydroxybenzanilide (99.6 grams, 0.434 mole) prepared from the method given in A, epichlorohydrin (804.57 grams, 8.70 moles), deionized water (69.96 grams, 8.0 percent by weight of the epichlorohydrin used) and isopropanol (433.23 grams, 35 percent by weight of the epichlorohydrin used) were added to a round bottom flask and heated to 65°C with stirring under a nitrogen atmosphere. After the temperature had reached 65°C, sodium hydroxide (31.31 grams, 0.78 mole) dissolved in deionized water (125.25 grams) was added dropwise over a one hour period so as to maintain the reaction temperature at 65°C. Fifteen minutes after completion of the aqueous sodium hydroxide addition, the stirring was stopped and the aqueous layer which separated from the reaction mixture was removed and discarded. Stirring was then resumed and a second solution of sodium hydroxide (13.92 grams, 0.35 mole) dissolved in deionized water (55.66 grams) was added over a 30 minute period so as to maintain the reaction temperature at 65°C. Fifteen minutes after completion of the second aqueous sodium hydroxide addition, stirring and heating were stopped and the reaction mixture was transferred to a separatory funnel. The aqueous layer which separated from the reaction mixture was removed and discarded. As the remaining organic layer cooled to room temperature, sufficient methylene chloride was added to keep the epoxy resin dissolved in solution. The cooled organic layer obtained was then washed four times with deionized water. The volume of deionized water used during each wash was approximately one half that of the organic layer. The washed organic layer was then rotary evaporated under vacuum at 125°C. The final product obtained after drying was an off-white crystalline solid (142.03 grams, yield = 95.8 percent based on 4,4'-dihydroxybenzanilide) which had a melting point of 180-185°C and an epoxide equivalent weight of 178.0.

### C. Characterization of the Diglycidyl Ether of 4,4'-Dihydroxybenzanilide for Liquid Crystal Character

A sample of the diglycidyl ether of 4,4'-dihydroxybenzanilide prepared in B was heated on a hot stage and viewed under an optical microscope (70X magnification) using a cross polarized light source.

Melting and clearing to an isotropic state was observed between 179°-185°C. Upon cooling from 185°C, the development of a birefringent phase was first observed at 165°C which was completed at 160°C. In this temperature range, the resin was still fluid. On further cooling, the resin was observed to crystallize at approximately 150°C.

Differential scanning calorimetry analysis of the diglycidyl ether of 4,4'-dihydroxybenzanilide at a heating rate of 20°C per minute showed a small endotherm (14 joules/gram) between 150°-170°C followed by a melt endotherm at 183°C. On cooling at 20°C per minute, a small broad exotherm was observed between 180° and 148°C followed by a larger exotherm beginning at 148°C (-50 joules/gram). These observed transitions change with repeated heating and cooling which was attributed to the slow self-cure of the diglycidyl ether of 4,4'-dihydroxybenzanilide at these temperatures.

To determine the characteristics of the diglycidyl ether of 4,4'-dihydroxybenzanilide when chain extended, 0.2504 grams of this resin containing 6000 ppm A-2 phosphonium catalyst was combined and mixed with 0.67 equivalent of 4,4'-dihydroxybenzanilide (0.1083 gram). This mixture was placed between two glass plates and then heated to 250°C where an isotropic film was produced. Upon cooling, a birefringent schlieren type texture was observed for this film when viewed under an optical microscope (70X magnification) using a cross polarized light source. These results indicate that a liquid crystal glass can be produced through the advancement and/or cure of the diglycidyl ether of 4,4'-dihydroxybenzanilide.

### D. Preparation of Cast Films of the Diglycidyl Ether of 4,4'-Dihydroxybenzanilide

Part of the diglycidyl ether of 4,4'-dihydroxybenzanilide prepared from B (4.3925 grams) was combined and mixed with an equivalent amount of a curing agent, sulfanilamide (1.0624 grams). This blend of solids was then placed in a 175°C convection oven. After melt has occurred (approximately 10 minutes) the resinous mixture was poured into a mold (dimensions = 7.5 inch x 0.5 inch x 0.021 inch; 190.5 mm x 12.7 mm x 0.53 mm) also located in the same convection oven. This mold was then placed in a mechanical press heated to 130°C. Pressure (approximately 2000 psi) was then applied to the mold within 5 minutes. After 1.5 hours at 130°C, the temperature of the press was increased and held for one hour at the following temperatures: 160°C, 180°C and 200°C. After one hour at 200°C, the temperature of the press was raised to 225°C where it was held for two hours before it was allowed to cool to room temperature. After cooling to room temperature an opaque casting was obtained from the mold. The flash from this casting, which results from excess resin being squeezed from the mold, exhibits a birefringent texture which was oriented in the direction of its flow formation. The apparent glass transition temperature for this polymer was 245°C as determined by thermal mechanical analysis which was 65°C higher than that obtained for the diglycidyl ether of bisphenol A in Comparative Experiment A-2. The average tensile strength and modulus for two cast films prepared using this procedure were 12,450 psi (standard deviation = 1460 psi) and 635 ksi (standard deviation = 3.5 ksi), respectively. These values represent a 41 and 23 percent improvement for these respective properties when compared to those obtained for the diglycidyl ether of bisphenol A in Comparative Experiment A-2. The results are reported in Table VI.

**TABLE VI**

| PROPERTIES FOR CASTED FILMS | | |
|---|---|---|
| PROPERTY | DIGLYCIDYL ETHER OF BISPHENOL A (Comparative Experiment A-2) | DIGLYCIDYL ETHER OF 4,4'-DIHYDROXYBENZANILIDE (Example 2-D) |
| Glass Transition Temperature, °C (Thermal Mechanical Analysis) | 180 | 245 (apparent) |
| Tensile Strength, | 360.74 MPa (8803 psi) | 85.9 MPa (12,450 psi) |
| Tensile Modulus, | 3553,5 MPa (515 ksi) | 4381,5 MPa (635 ksi) |

### Example 3

### A. Synthesis of 4,4'-Dihydroxy-2,2'-Dimethylazoxybenzene

3-Methyl-4-nitrophenol (15.3 grams, 0.10 mole) in anhydrous tetrahydrofuran (100 milliliters) was added dropwise to a vigorously stirred, refluxing suspension of excess lithium aluminum hydride (6.0 grams) in anhydrous tetrahydrofuran (100 milliliters). After completion of the addition, reflux was maintained for an additional 24 hours. The recovered mixture was poured over crushed ice then extracted with diethyl ether. The ether extract was dried over anhydrous magnesium sulfate followed by rotary evaporation under vacuum to provide a wine red solid identified as 4,4'-dihydroxy-2,2'-dimethylazobenzene by nuclear magnetic resonance spectroscopy. Oxidation of the recovered azo compound was accomplished by dissolution in tetrahydrofuran followed by addition of a 10 mole percent excess of 32 percent peracetic acid at 25°C. Two hours after addition of the peracetic acid, the solution was poured into a mixture of saturated sodium bisulfite (50 milliliters) and saturated sodium bicarbonate (50 milliliters). The precipitated solid was filtered from the aqueous mixture then recrystallized from deionized water to provide 4,4'-dihydroxy-2,2'-dimethylazoxybenzene (11.0 grams).

### B. Epoxidation of 4,4'-Dihydroxy-2,2'-Dimethylazoxybenzene

4,4'-Dihydroxy-2,2'-dimethylazoxybenzene (9.0 grams, 0.07 hydroxyl equivalent), epichlorohydrin (64.5 grams, 0.7 mole), deionized water (2.8 grams, 8.0 percent by weight of the epichlorohydrin used) and isopropanol (17.4 grams, 35 percent by weight of the epichlorohydrin used) were added to a reactor and heated to 55°C with stirring under a nitrogen atmosphere. Once the 55°C reaction temperature was achieved, sodium hydroxide (2.51 grams, 0.063 mole) dissolved in deionized water (10.0 grams) was added dropwise to the reactor over a 40 minute period in order to maintain the reaction temperature at 55°C. Ten minutes after completion of the aqueous sodium hydroxide addition, the stirring was stopped and the aqueous layer which separated from the reaction mixture was pipetted off and discarded. Stirring was resumed and after a total of twelve minutes following completion of the initial aqueous sodium hydroxide addition, a second solution of sodium hydroxide (1.12 grams, 0.028 mole) dissolved in deionized water (4.5 grams) was added to the reactor over a twenty minute period in order to maintain the 55°C reaction temperature. Fifteen minutes after completion of the aqueous sodium hydroxide addition, the recovered reaction mixture was added to a separatory funnel and washed with 300 milliliters of deionized water. The separated organic layer was washed a second time (300 milliliters deionized water), recovered and then rotary evaporated under vacuum for 60 minutes at 100°C. The product was recovered as a crystalline mustard yellow colored solid with an epoxide equivalent weight of 202.26 (uncorrected).

### C. Characterization of the Diglycidyl Ether of 4,4'-Dihydroxy-2,2'-Dimethylazoxybenzene for Liquid Crystal Character

A sample of the diglycidyl ether of 4,4'-dihydroxy-2,2'-dimethylazoxybenzene prepared in B was heated on a hot stage and viewed under an optical microscope (70X magnification) using a cross polarized light source. Softening of this resin was first observed at 50°C. On further heating, a melt was obtained at 86°C which contained birefringent particles. These birefringent particles began to disappear at 96°C and a totally isotropic melt was observed at 102°C. On cooling the resin from 102°C to room temperature, no liquid crystal textures were observed.

The diglycidyl ether of 4,4'-dihydroxy-2,2'-dimethylazoxybenzene prepared from B (0.1200 gram) was combined and mixed with an equivalent amount of sulfanilamide (0.0255 gram). Sulfanilamide was chosen in this analysis as it is an epoxy curing agent which promotes a degree of linear advancement before final crosslinking. A sample of this curable composition was heated on a hot stage and viewed under an optical microscope (70X magnification) using a cross polarized light source. At 170°C, an isotropic melt was obtained which set after approximately 15 minutes at this temperature. After one hour at 170°C, the temperature was increased to 200°C where it was held for 40 minutes before cooling to room temperature. Upon cooling to room temperature, a birefringent schlieren type pattern was observed which indicates the formation of a polymer which was a liquid crystalline glass.

### Example 4

### A. Synthesis of 4,4'-Dihydroxy-α-Methylstilbene

Phenol (376.144 grams, 4.0 moles), chloroacetone (192.77 grams, 2.0 moles as chloroacetone) and methylene chloride (300 grams) were added to a reactor and cooled to -10°C with stirring. The chloroacetone used was a commercial grade containing 96 percent chloroacetone. Concentrated sulfuric acid (196.16 grams, 2.0 mole) was added dropwise to the stirred solution over a thirty seven minute period and so as to maintain the reaction temperature between -10 and -11°C. After 143 minutes of post reaction between a -10 to -11°C temperature range, the viscous, orange colored oil product was mixed with iced deionized water (500 milliliters). The oil product was separated then washed with a second portion (500 milliliters) of deionized water. After separation, the recovered oil product was added to a 2 liter beaker along with ethanol (250 milliliters) and stirred to provide a solution. Deionized water (250 milliliters) was added to the stirred solution and heating commenced. As the temperature of the mixture increased, the stirred mixture began to clear. Each time clearing was observed, sufficient deionized water was added to induce cloudiness, followed by continuation of the mixing and heating. Once the temperature reached 70°C, massive precipitation of white crystalline plates occurred and was followed by immediate coalescence of the precipitated product to an oil. The oil layer was recovered by decantation of the water layer and ethanol (250 milliliters) was added. Deionized water was again added to the stirred solution as heating commenced, in an amount sufficient to induce cloudiness each time clearing was observed. Once the temperature reached 90°C, a massive precipitation of white crystalline plates again occurred. At this time, stirring was stopped and the crystalline slurry was chilled to 4°C and held therein for 12 hours. The crystalline product was recovered by filtration of the chilled crystalline slurry and combined with deionized water (800 milliliters), then stirred with heating to 90°C. After maintaining the stirred slurry at 90°C for five minutes, the crystalline product was recovered by filtration. The crystalline product was again combined with deionized water (800 milliliters), then stirred with heating to 90°C. After maintaining the stirred slurry at 90°C for five minutes, the crystalline product was recovered by filtration and then dried in a vacuum oven at 100°C and 666,4 Pa (5 mm Hg) to a constant weight of 190.0 grams of light tan colored crystalline solid. Proton magnetic resonance spectroscopy and infrared spectrophotometric analysis confirmed the product structure.

### B. Epoxidation of 4,4'-Dihydroxy-α-methylstilbene

4,4'-Dihdroxy-α-methylstilbene (152.73 grams, 1.35 hydroxyl equivalents) from A above, epichlorohydrin (624.58 grams, 6.75 moles), deionized water (54.31 grams, 8.0 percent by weight of the epichlorohydrin used) and isopropanol (336.31 grams, 35 percent by weight of the epichlorohydrin used) were added to a reactor and heated to 55°C with stirring under a nitrogen atmosphere. Once the 55°C reaction temperature was achieved, sodium hydroxide (48.6 grams, 1.22 moles) dissolved in deionized water (194.4 grams) was added dropwise to the reactor over a 45 minute period and so as to maintain reaction temperature between 55 and 59°C. Ten minutes after completion of the aqueous sodium hydroxide addition, the stirring was stopped and the aqueous layer which separated from the reaction mixture was pipetted off and discarded. Stirring was resumed and after a total of twenty minutes following completion of the initial aqueous sodium hydroxide addition, a second solution of sodium hydroxide (21.6 grams, 0.54 mole) dissolved in deionized water (86.4 grams) was added to the reactor over a twenty minute period and so as to maintain the 55°C reaction temperature. Fifteen minutes after completion of the aqueous sodium hydroxide addition, the recovered reaction mixture was added to a separatory funnel and washed with 750 milliliters of deionized water. The separated organic layer was washed a second time (750 milliliters deionized water), recovered and then rotary evaporated under vacuum for 45 minutes at 110°C then 30 minutes at 130°C. The product was recovered (218.6 grams) as a crystalline solid with an epoxide equivalent weight of 183.33.

### C. Characterization of Liquid Crystallinity in the Diglycidyl Ether of 4,4'-Dihydroxy-α-Methylstilbene

A portion (13.12 milligrams) of the diglycidyl ether of 4,4'-dihydroxy-α-methylstilbene from B above was analyzed by differential scanning calorimetry using a heating rate of 10°C per minute and a temperature range of 30 to 150°C. The following results were obtained:

| Cycle Designation | Observed Transition Temperatures (°C) midpoint/range | Enthalpy (J/g) | Comments |
|---|---|---|---|
| First heating (30 to 150°C) | 127/101-138 | 72.7 | single endotherm |
| First cooling (150 to 30°C) | 89/92-78 | 1.8 | single exotherm |
| | 44/54-36 | 21.2 | single exotherm |
| Second heating (30 to 150°C) | 82/72-96 | 26.0 | single exotherm |
| | 126/96-138 | 61.9 | single endotherm |
| Second cooling (150 to 30°C) | 89/92-73 | 3.0 | single exotherm |
| | 44/54-36 | 21.8 | single exotherm |

Analysis of the diglycidyl ether via crosspolarized light microscopy was completed using a microscope equipped with a programmable hot stage using a heating rate of 10°C per minute. The following results were obtained:

| Cycle Designation | Observed Transition Temperatures (°C) | Comments |
|---|---|---|
| First heating (25 to 136°C) | 30 | Birefringent crystalline solid. |
| | 107 | First fluidity noted, birefringent crystals moving in an isotropic fluid. |
| | 135 | Isotropization completed. |
| First cooling (136 to 30°C) | 94 | First mobile nematic droplets observed. |
| | 56.5 | Crystallizes. |
| Second heating (30 to 136°C) | 77 | First fluidity noted. |
| | 106 | Birefringent crystals moving in an isotropic fluid. |
| | 132 | Isotropization completed. |
| Second cooling (129 to 30°C) | 92 | First mobile nematic droplets observed. |
| | 57 | Crystallizes. |

The diglycidyl ether is a monotropic liquid crystal with a nematic texture. The nematic fluid gave opalescence when stirred between the 94 and 56.5°C temperatures of the first cooling cycle.

### D. Characterization of Liquid Crystallinity in the Phenoxy Resin Prepared via in situ Reaction of 4,4'-Dihydroxy-α-methylstilbene and the Diglycidyl Ether 4,4'-Dihydroxy-α-methylstilbene

A diglycidyl ether of 4,4'-dihydroxy-α-methylstilbene (0.25 gram, 0.0014 epoxide equivalent) prepared using the method of B above and having an epoxide equivalent weight of 176.81, 4,4'-dihydroxy-α-methylstilbene (0.16 gram, 0.0014 hydroxyl equivalent) prepared using the method of A above and acetone (20 milliliters) to which tetrabutylphosphonium acetate·acetic acid complex (70 percent solids in methanol) (0.0075 gram) had been added was mixed to provide a solution. The resultant solution was evaporated to provide a dry solid which was then ground to a fine homogeneous powder. A portion of the powder on a microscope slide was analyzed via crosspolarized light microscopy using a microscope equipped with a programmable hot stage and using a heating rate of 10°C per minute to 131°C then held therein. At 131°C, a totally isotropic fluid formed. After one minute at 131°C, a birefringent phase with a rodlike appearance was observed. After two minutes at 131°C, the birefringent, rodlike appearance increased and stir opalescence was present. After five minutes at 131°C, the product became an opaque, birefringent solid. Heating of the opaque, birefringent solid to 250°C produced no further changes in its appearance. Upon cooling to 25°C, the solid product retained its opaque and birefringent appearance.

### E. Preparation of Phenoxy Resin of 4,4'-Dihydroxy-α-Methylstilbene and the Diglycidyl Ether of 4,4'-Dihydroxy-α-Methylstilbene and Solvent Borne Coating Thereof

A portion (5.6566 grams, 0.05 hydroxyl equivalent) of 4,4'-dihydroxy-α-methylstilbene from A above, a portion (9.1666 grams, 0.05 epoxide equivalent) of the diglycidyl ether of 4,4'-dihydroxy-α-methylstilbene from B above and cyclohexanone (50.0 grams) were added to a reactor and heated with stirring under a nitrogen atmosphere to provide a 90°C solution. Once the 90°C reaction temperature was achieved, ethyltriphenyl-phosphonium acetate·acetic acid complex (70 percent solids in methanol) (0.0297 gram, 0.20 percent by weight of the diphenol and diglycidyl ether reactants used) was added to the reactor and heating continued to 130°C. After eight hours at the 130°C temperature, the reaction product was recovered as a transparent, viscous solution. Cyclohexanone was evaporated from the product solution to provide 33.3 percent by weight phenoxy resin (14.82 grams) in 66.7 percent by weight cyclohexanone (29.7 grams). This solution was applied to the surface of a (4 inch by 12 inch by 0.32 inch) 101 mm x 304 mm x 8.1 mm unpolished cold rolled steel panel which had been washed with methylene chloride using a number 40 drawdown bar. The coated panel was allowed to dry for twelve hours at room temperature (25°C) to provide a smooth opaque coating which was free of flaws. Further drying at 100°C was completed for eight hours, then a sample of the coating was scraped off and examined via crosspolarized light microscopy demonstrating birefringence. A portion of the 1 mil thick coated panel was tested for flexibility via the T-bend test using a standard method (ASTM D 4145-83). The temperature at which the specimens were bent was 25°C, with all bends made perpendicular to the direction of the coating drawdown. After the application and removal of the specified pressure sensitive tape to the bent surface, the coating in the bend region was treated for 15 seconds with acidified copper sulfate. After rinsing to remove the acidified copper sulfate, the blotted surface was examined via optical microscopy for defects. The coating failed 0T as evidenced by the penetration of acidified copper sulfate to etch the metal surface, but consistently passed 1T (no penetration of acidified copper sulfate).

### Comparative Experiment B

### Preparation of a Solvent Borne Coating or The Phenoxy Resin of 4,4'-Isopropylidenediphenol and the Diglycidyl Ether of 4,4'-Isopropylidenediphenol

Cyclohexanone (66.7 percent weight, 29.7 grams) and a commercial grade phenoxy resin prepared from 4,4'-isopropylidenediphenol and the diglycidyl ether of 4,4'-isopropylidenediphenol (33.3 percent weight, 14.82 grams) were combined to form a solution. This solution was applied to the surface of a (4 inch x 12 inch x 0.32 inch) 101 mm x 304 mm x 8.1 mm unpolished cold rolled steel panel which had been washed with methylene chloride using a number 40 drawdown bar. The coated panel was allowed to dry for twelve hours at room temperature (25°C) to provide a smooth transparent coating which was free of flaws. Further drying at 100°C was completed for eight hours, then a sample of the coating was scraped off and examined via crosspolarized light microscopy demonstrating a total lack of birefringence. A portion of the 1 mil thick coated panel was tested for flexibility via the T-bend test using a standard method (ASTM D 4145-83). The temperature at which the specimens were bent was 25°C, with all bends made perpendicular to the direction of the coating drawdown. After the application and removal of the specified pressure sensitive tape to the bent surface, the coating in the bend region was treated for 15 seconds with acidified copper sulfate. After rinsing to remove the acidified copper sulfate, the blotted surface was examined via optical microscopy for defects. The coating tailed 0T as evidenced by the penetration of acidified copper sulfate to etch the metal surface, sometimes passed 1T, but consistently passed 2T (no penetration of acidified copper sulfate).

## Claims

1. An epoxy resin containing one or more mesogenic or rodlike moieties, said epoxy resins represented by the following Formula I: 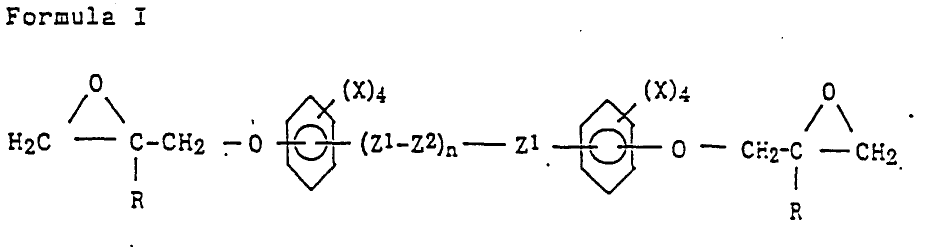 wherein at least 80 percent of the -(Z¹-Z²)ₙ-Z¹-linkages and the glycidyl ether groups are in the para position with respect to each other; each R and R¹ is independently hydrogen or an aliphatic hydrocarbon group having from 1 to 4 carbon atoms; each X is independently hydrogen, a hydrocarbyl or hydrocarbyloxy group having from 1 to 12 carbon atoms, a halogen atom, -NO₂, or -C≡N; each Z¹ is independently -CR¹=CR¹-, -CR¹=CR¹-CR¹=CR¹-, -CR¹=N-N=CR¹-, -CR¹=CR¹-CO-O-CH₂-, -CR¹=CR¹-CO-O-CH₂-CH₂-, -CH₂-O-CO-CR¹=CR¹-, -CH₂-CH₂-O-CO-CR¹=CR¹-, -CR¹=CR¹-CO-O-, -O-CO-CR¹=CR¹-, -CO-NH-, -NH-CO-, -CO-NH-NH-CO-, -C≡C-, -C≡C-C≡C-, -CO-S-, -S-CO-, a direct single bond when n ≧ 1, 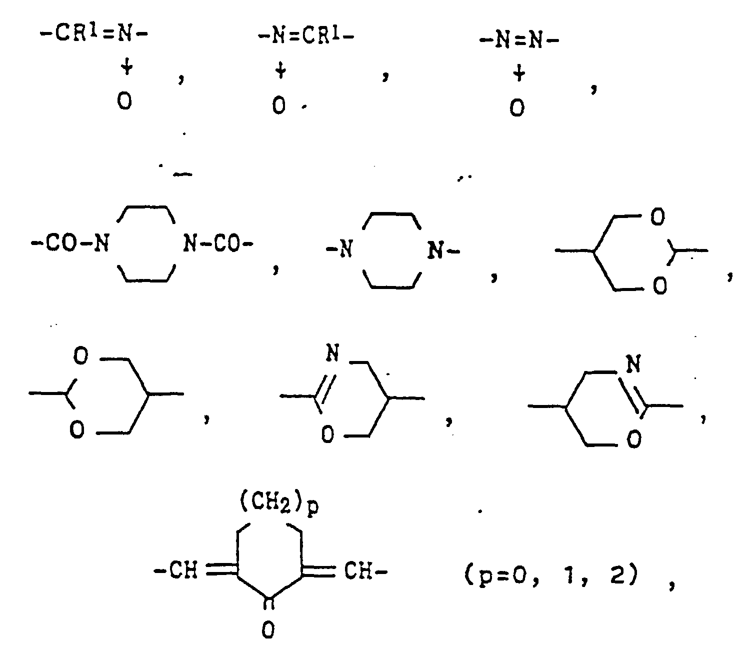 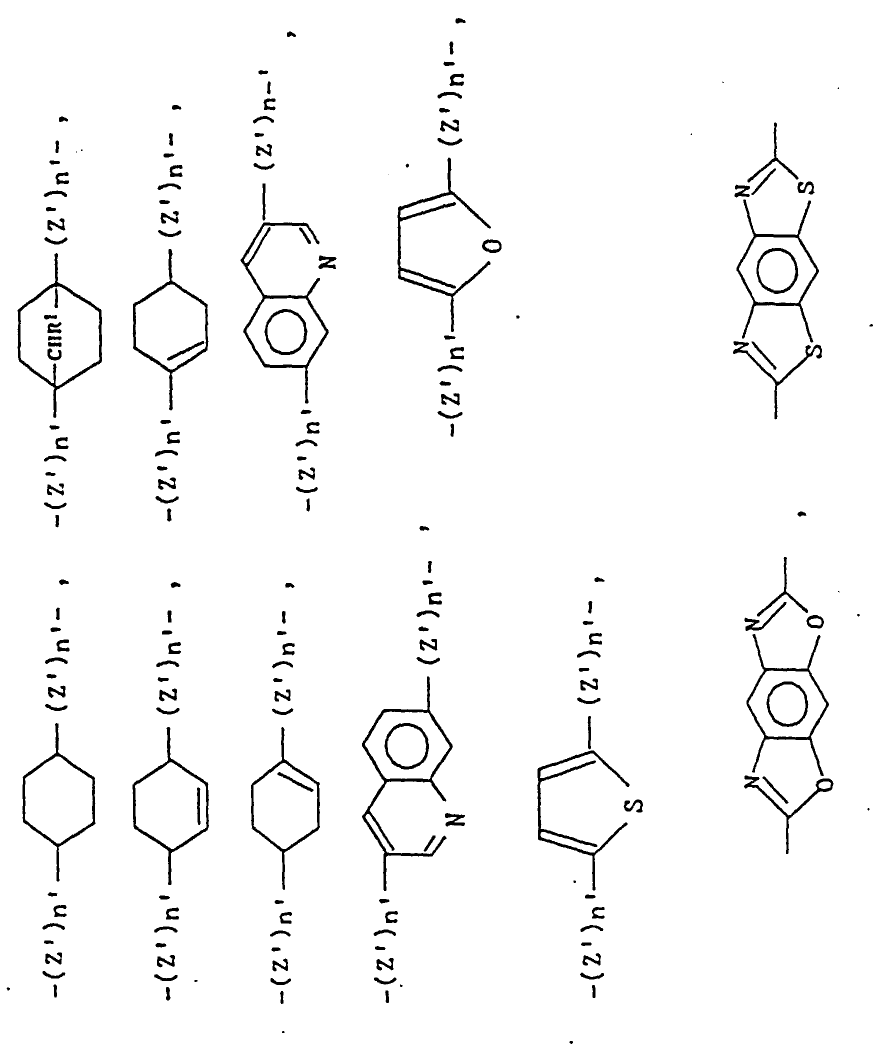 Z¹ can also independently be 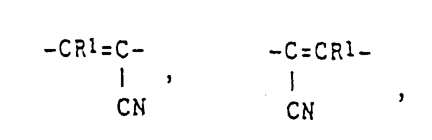 -CR¹=N-, -N=CR¹-, -CO-CR¹=CR¹-, -CR¹=CR¹-CO-, -CO-O-, and -O-CO- when Z² is not a benzene ring and n≠0; Z² is a group represented by a cyclic or bicyclic ring system containing from 5 to 12 carbon atoms and may be cycloaliphatic, polycycloaliphatic, aromatic or a combination thereof; Z' is independently a -CO-, -O-CO-, -CO-O-, -CO-NR¹-, or -NR¹-CO- group; n is zero to 2; and n' is zero or 1, or represented by the following Formula II: 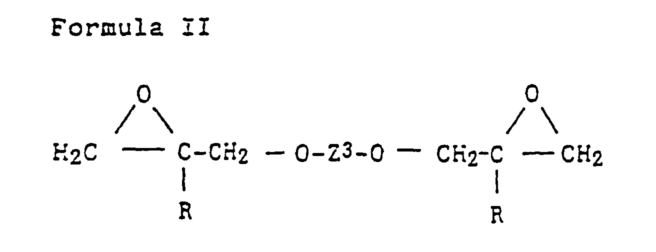 wherein Z³ is 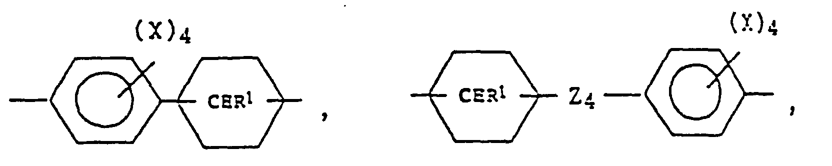 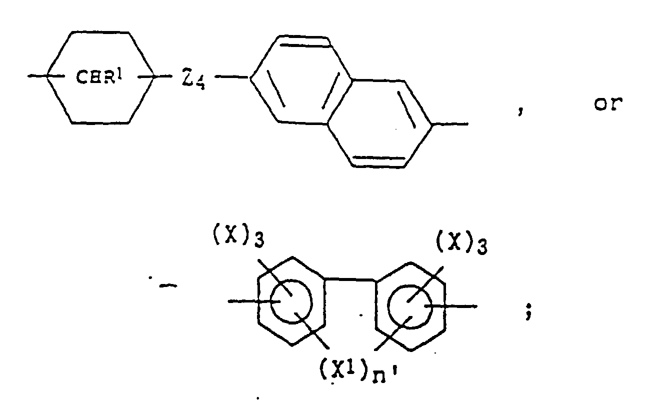 and Z⁴ is -CO-O-, -O-CO-, -NR¹-CO- or -CO-NR¹-; and X¹ is a hydrocarbyl group having from 1 to 10 carbon atoms which can contain one or more heteroatoms selected from N, O, and S and may be saturated or unsaturated and R¹, X and R are as defined above.

2. The diglycidyl ether of 4,4'-dihydroxy-α-methylstilbene.

3. The diglycidyl ether of 4,4'-dihydroxybenzanilide.

4. The diglycidyl ether of 4,4'-dihydroxy-2,2'-dimethylazoxybenzene.

5. An advanced epoxy resin composition prepared by reacting (A) one or more of the epoxy resins or compositions of Claims 1, 2, 3, or 4 with (B) at least one compound having an average of more than one active hydrogen atom per molecule; wherein components (A) and (B) are employed in amounts which provide a ratio of active hydrogen atoms per epoxide group of from 0.01:1 to 0.95:1.

6. A thermoplastic resin composition prepared by advancement reaction of (A) one or more of the epoxy resins or compositions of Claims 1, 2, 3, 4 or 5 with (B) at least one compound having an average of more than one active hydrogen atom per molecule; wherein components (A) and (B) are employed in amounts which provide a ratio of active hydrogen atoms per epoxide group of from 0.96:1 to 1.05:1.

7. An epoxy resin composition of claims 1, 2, 3, 4, 5, or 6 wherein the mesogenic or rodlike moieties are oriented.

8. An epoxy resin composition of Claim 7 wherein said orientation is accomplished by means of an electric field or magnetic field or shear stress.

9. A blend comprising (A) one or more epoxy-containing resins or compounds containing one or more mesogenic or rodlike moieties of Claims 1, 2, 3, 4, and (B) one or more polyepoxides which are substantially free of mesogenic or rodlike moieties; wherein component (A) is present in an amount of from 1 to 99 percent by weight of the combined weight of components (A) and (B) and component (B) is present in an amount of from 99 to 1 percent by weight of the combined weight of components (A) and (B).

10. A blend comprising (A) one or more epoxy-containing resins or compounds containing one or more mesogenic or rodlike moieties of Claim 5 and (B) one or more polyepoxides not containing mesogenic or rodlike moieties; wherein component (A) is present in an amount of from 1 to 99 percent by weight of the combined weight of components (A) and (B) and component (B) is present in an amount of from 99 to 1 percent by weight of the combined weight of components (A) and (B).

11. A curable composition comprising one or more epoxy resins, compositions, or blends of anyone of Claims 1 to 5 9 or 10 and a curing quantity of at least one curing agent therefor.

12. A curable composition comprising (A) one or more of the epoxy resins or compositions of Claims 1 to 5; (B) one or more of
a monoepoxide containing one or more mesogenic or rodlike moieties, said monoepoxide being represented by the following Formula III: 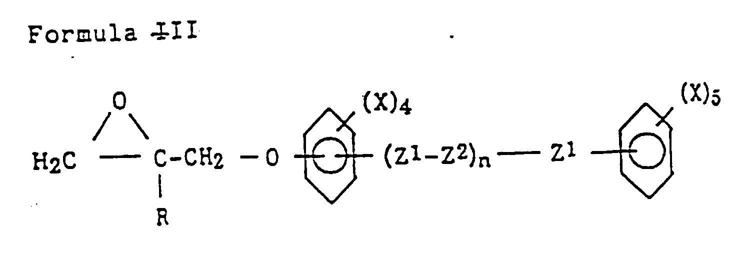 wherein at least 80 percent of the -(Z¹-Z²)ₙ-Z¹-linkages and the glycidyl ether groups are in the para position with respect to each other; each R and R¹ is independently hydrogen or an aliphatic hydrocarbon group having from 1 to 4 carbon atoms; each X is independently hydrogen, a hydrocarbyl or hydrocarbyloxy group having from 1 to 12 carbon atoms, a halogen atom, -NO₂, or -C≡N; each Z¹ is independently -CR¹=CR¹-, -CR¹=CR¹-CR¹=CR¹-, -CR¹=N-N=CR¹-, -CR¹=CR¹-CO-O-CH₂-, -CR¹=CR¹-CO-O-CH₂-CH₂-, -CH₂-O-CO-CR¹=CR¹-, -CH₂-CH₂-O-CO-CR¹=CR¹-, -CR¹=CR¹-CO-O-, -O-CO-CR¹=CR¹-, -N=N-, -CO-NH-, -NH-CO-, -CO-NH-NH-CO-, -C≡C-, -C≡C-C≡C-, -CO-S-, -S-CO-, -CR¹=N-, -N=CR¹-, -CO-O-, -O-CO-, a direct single bond when n≧1, 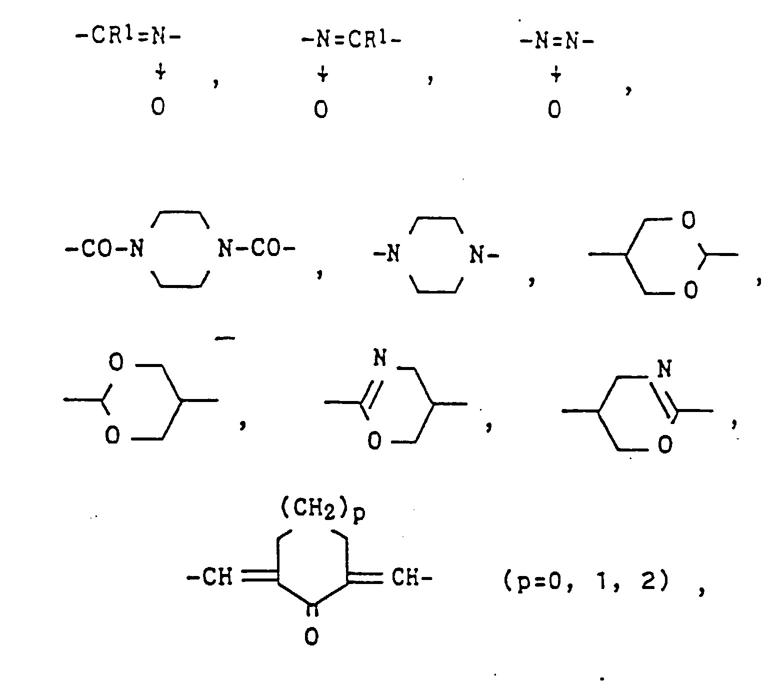 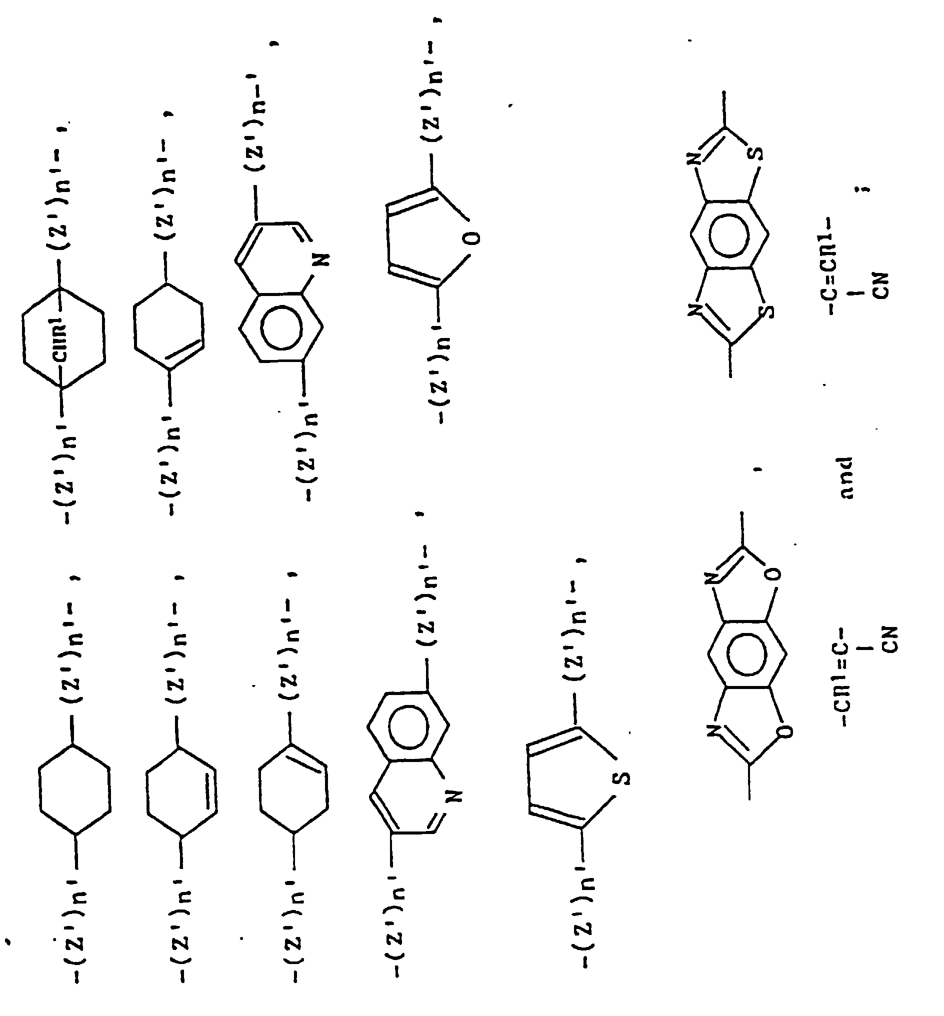 Z² is a group represented by a cyclic or bicyclic ring system containing from 5 to 12 carbon atoms; or a monoepoxide being represented by the following Formula IV: 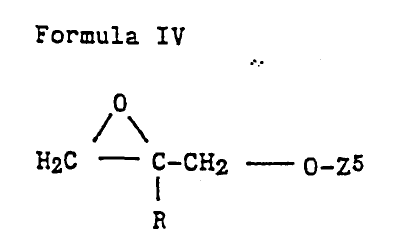 wherein Z⁵ is 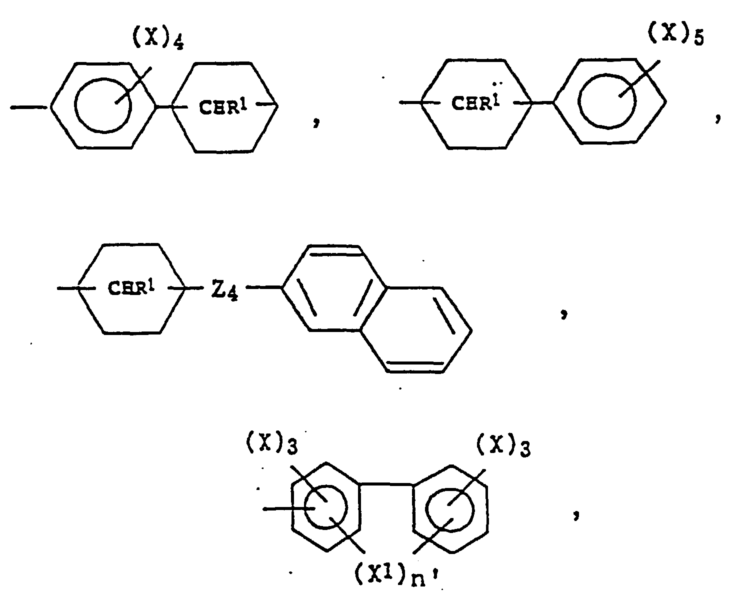 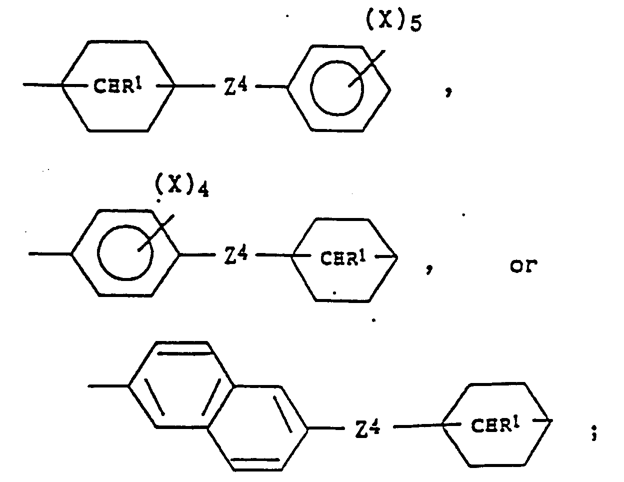 Z⁴ is -CO-O-, -O-CO-, -NR¹-CO- or -CO-NR¹-;
and (C) a curing amount of at least one curing agent therefor; wherein component (A) is present in an amount of from 1 to 99 percent by weight based upon the combined weight of components (A) and (B) and component (B) is present in an amount of from 99 to 1 percent by weight based upon the combined weight of components (A) and (B).

13. The product resulting from curing any one of the compositions of Claims 11 or 12.

14. The product of Claim 13 resulting from the application of an electric field, a magnetic field or a shear stress prior to and/or during cure.

## Patentansprüche

1. Epoxyharz, enthaltend eine oder mehrere mesogene oder stabförmige Einheiten, wobei diese Epoxyharze durch die folgende Formel I wiedergegeben werden: 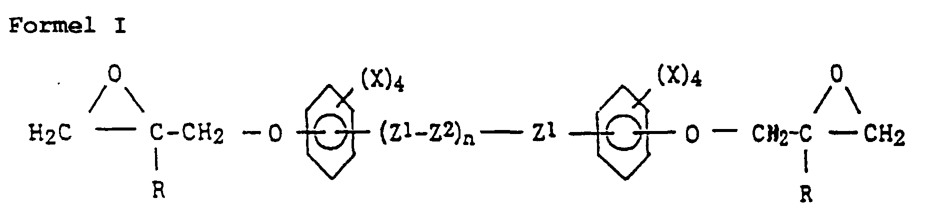 worin wenigstens 80 % der Bindungen -(Z¹-Z²)ₙ-Z¹- und die Glycidylethergruppen in der para-Stellung jeweils zueinander stehen; jedes R und R¹ unabhängig Wasserstoff oder eine aliphatische Kohlenwasserstoffgruppe mit 1 bis 4 Kohlenstoffatomen ist; jedes X unabhängig Wasserstoff, eine Hydrocarbyl- oder Hydrocarbyloxygruppe mit 1 bis 12 Kohlenstoffatomen, ein Halogenatom, -NO₂ oder -C≡N ist; jedes Z¹ unabhängig -CR¹=CR¹-, -CR¹=CR¹-CR¹=CR¹-, -CR¹=N-N=CR¹-, -CR¹=CR¹-CO-O-CH₂-, -CR¹=CR¹-CO-O-CH₂-CH₂-, -CH₂-O-CO-CR¹=CR¹-, -CH₂-CH₂-O-CO-CR¹=CR¹-, -CR¹=CR¹-CO-O-, -O-CO-CR¹=CR¹-, -CO-NH-, -NH-CO-, -CO-NH-NH-CO-, -C≡C-, -C≡C-C≡C-, -CO-S-, -S-CO-, eine direkte Einzelbindung, falls n ≥ 1, 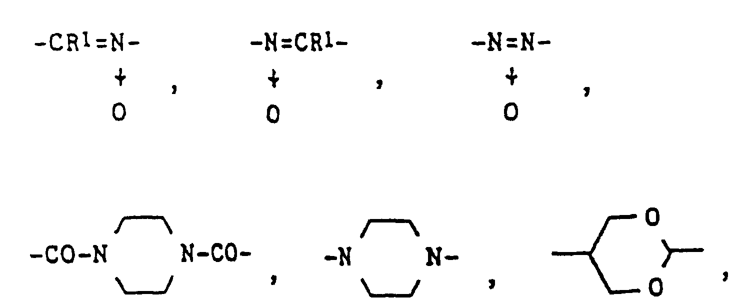 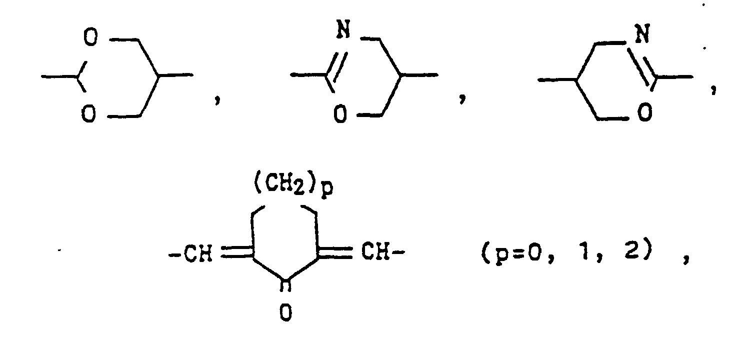 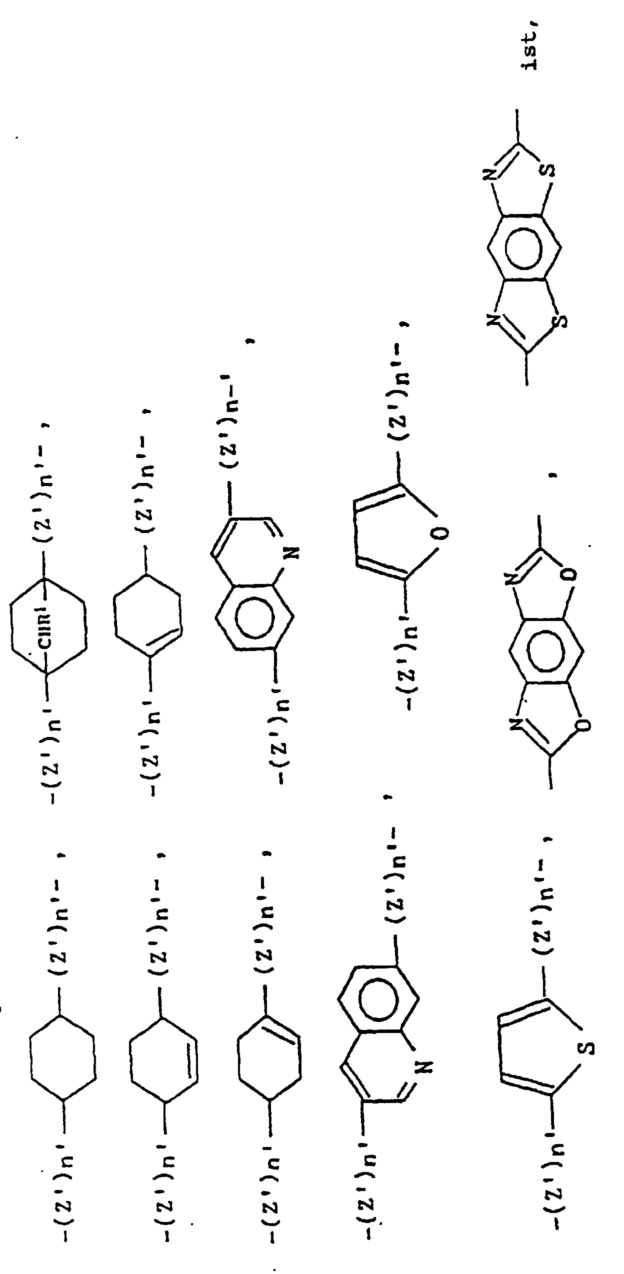 Z¹ ebenfalls unabhängig sein kann 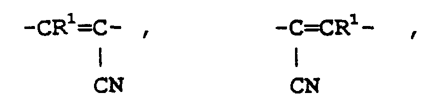 -CR¹=N-, -N=CR¹-, -CO-CR¹=CR¹-, -CR¹=CR¹-CO-, -CO-O- und -O-CO-, falls Z² nicht ein Benzolring ist und n ≠ 0; Z² eine Gruppe ist, die durch ein cyclisches oder bicyclisches Ringsystem wiedergegeben wird, das 5 bis 12 Kohlenstoffatome enthält und cycloaliphatisch, polycycloaliphatisch, aromatisch oder eine Kombination hiervon sein kann; Z' unabhängig eine Gruppe -CO-, -O-CO-, -CO-O-, -CO-NR¹- oder -NR¹-CO- ist; n = 0 null bis 2 ist und n' = null oder 1 ist, oder durch die folgende Formel II wiedergegeben wird: 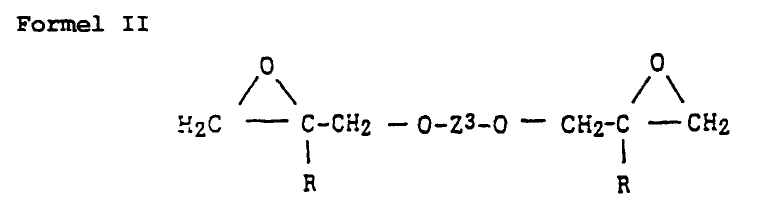 worin Z³ ist 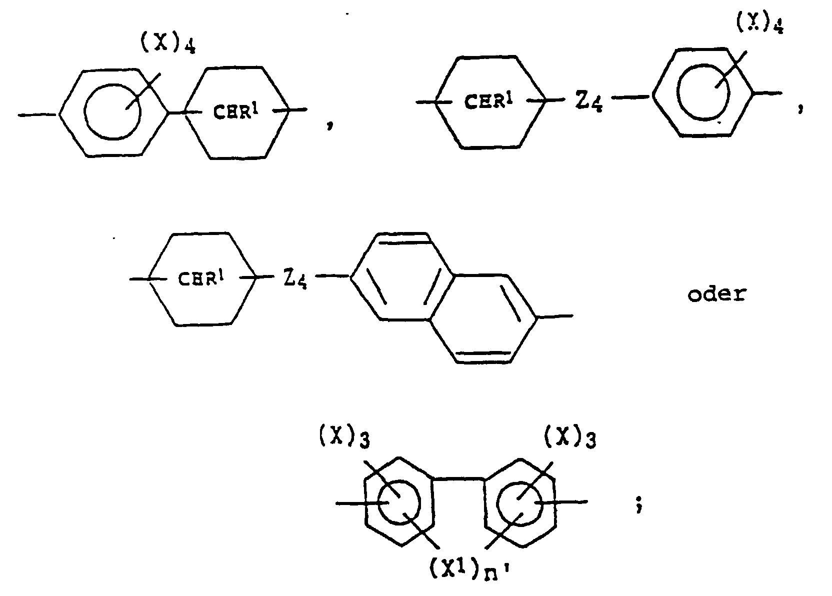 - und Z⁴ -CO-O-, -O-CO-, -NR¹-CO- oder -CO-NR¹- ist; und X¹ eine Hydrocarbylgruppe mit 1 bis 10 Kohlenstoffatomen ist, die ein oder mehrere Heteroatome, ausgewählt aus N, O und S, enthalten kann und gesättigt oder ungesättigt sein kann; und R¹, X und R wie oben definiert sind.

2. Der Diglycidylether von 4,4'-Dihydroxy-α-methylstilben.

3. Der Diglycidylether von 4,4'-Dihydroxybenzanilid.

4. Der Diglycidylether von 4,4'-Dihydroxy-2,2'-dimethylazoxybenzol.

5. Verlängerte Epoxyharzzusammensetzung, hergestellt durch Umsetzung von (A) einem oder mehreren Epoxyharzen oder -zusammensetzungen der Ansprüche 1, 2, 3 oder 4 mit (B) wenigstens einer Verbindung, die einen Durchschnitt von mehr als einem aktiven Wasserstoffatom pro Molekül besitzt, worin die Komponenten (A) und (B) in Mengen eingesetzt werden, die ein Verhältnis von aktiven Wasserstoffatomen pro Epoxidgruppe von 0,01:1 bis 0,95:1 ergeben.

6. Thermoplastische Epoxyharzzusammensetzung, hergestellt durch Verlängerungsreaktion von (A) einem oder mehreren Epoxyharzen oder -zusammensetzungen der Ansprüche 1, 2, 3, 4 oder 5 mit (B) wenigstens einer Verbindung, die einen Durchschnitt von mehr als einem aktiven Wasserstoffatom pro Molekül besitzt, worin die Komponenten (A) und (B) in Mengen eingesetzt werden, die ein Verhältnis von aktiven Wasserstoffatomen pro Epoxidgruppe von 0,96:1 bis 1,05:1 ergeben.

7. Epoxyharzzusammensetzung nach Anspruch 1, 2, 3 ,4, 5 oder 6, in welcher die mesogenen oder stabförmigen Einheiten orientiert sind.

8. Epoxyharzzusammensetzung nach Anspruch 7, in welcher diese Orientierung mittels eines elektrischen Feldes oder magnetischen Feldes oder durch Scherspannung herbeigeführt wurde.

9. Mischung, umfassend (A) ein oder mehrere epoxyhaltige Harze oder Verbindungen, enthaltend eine oder mehrere mesogene oder stabförmige Einheiten, von Anspruch 1, 2, 3, 4 und (B) ein oder mehrere Polyepoxide, die im wesentlichen frei von mesogenen oder stabförmigen Einheiten sind, worin die Komponente (A) in einer Menge von 1 bis 99 Gew.-% des kombinierten Gewichtes der Komponenten (A) und (B) vorhanden ist und die Komponente (B) in einer Menge von 99 bis 1 Gew.-% des kombinierten Gewichtes der Komponenten (A) und (B) vorhanden ist.

10. Mischung, umfassend (A) ein oder mehrere epoxyhaltige Harze oder Verbindungen, enthaltend eine oder mehrere mesogene oder stabförmige Einheiten, von Anspruch 5 und (B) ein oder mehrere Polyepoxide, die keine mesogenen oder stabförmigen Einheiten enthalten, worin die Komponente (A) in einer Menge von 1 bis 99 Gew.-% des kombinierten Gewichtes der Komponenten (A) und (B) vorhanden ist und die Komponente (B) in einer Menge von 99 bis 1 Gew.-% des kombinierten Gewichtes der Komponenten (A) und (B) vorhanden ist.

11. Aushärtbare Zusammensetzung, umfassend ein oder mehrere Epoxyharze, -zusammensetzungen oder -mischungen von einem der Ansprüche 1 bis 5, 9 oder 10 und eine aushärtende Menge wenigstens eines Härters hierfür.

12. Aushärtbare Zusammensetzung, umfassend
(A) ein oder mehrere der Epoxyharze oder -zusammensetzungen von Anspruch 1 bis 5;
(B) ein oder mehrere eines Monoepoxids, das eine oder mehrere mesogene oder stabförmige Einheiten enthält, wobei dieses Monoepoxid durch die folgende Formel III wiedergegeben wird: 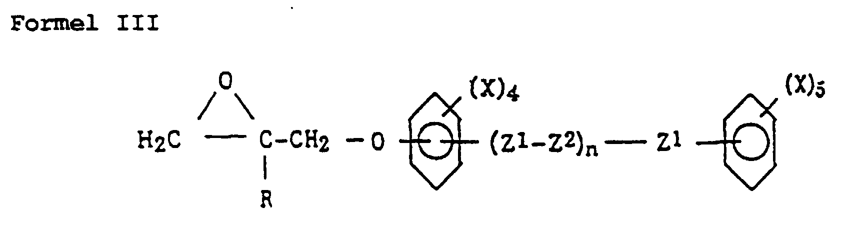 worin wenigstens 80 % der Bindungen -(Z¹-Z²)ₙ-Z¹- und die Glycidylethergruppen in der para-Stellung jeweils zueinander stehen; jedes R und R¹ unabhängig Wasserstoff oder eine aliphatische Kohlenwasserstoffgruppe mit 1 bis 4 Kohlenstoffatomen ist; jedes X unabhängig Wasserstoff, eine Hydrocarbyl- oder Hydrocarbylozygruppe mit 1 bis 12 Kohlenstoffatomen, ein Halogenatom, -NO₂ oder -C≡N ist; jedes Z¹ unabhängig -CR¹=CR¹-, -CR¹=CR¹-CR¹=CR¹-, -CR¹=N-N=CR¹-, -CR¹=CR¹-CO-O-CH₂-, -CR¹=CR¹-CO-O-CH₂-CH₂-, -CH₂-O-CO-CR¹=CR¹-, -CH₂-CH₂-O-CO-CR¹=CR¹-, -CR¹=CR¹-CO-O-, -O-CO-CR¹=CR¹-, -N=N-, -CO-NH-, -NH-CO-, -CO-NH-NH-CO-, -C≡C-, -C≡C-C≡C-, -CO-S-, -S-CO-, -CR¹=N-, -N=CR¹-, -CO-O-, -O-CO-, eine direkte Einzelbindung, falls n ≥ 1, 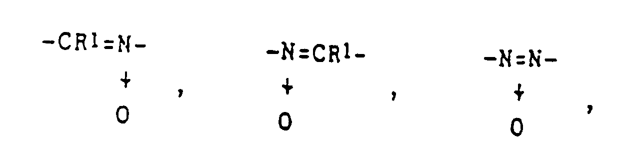 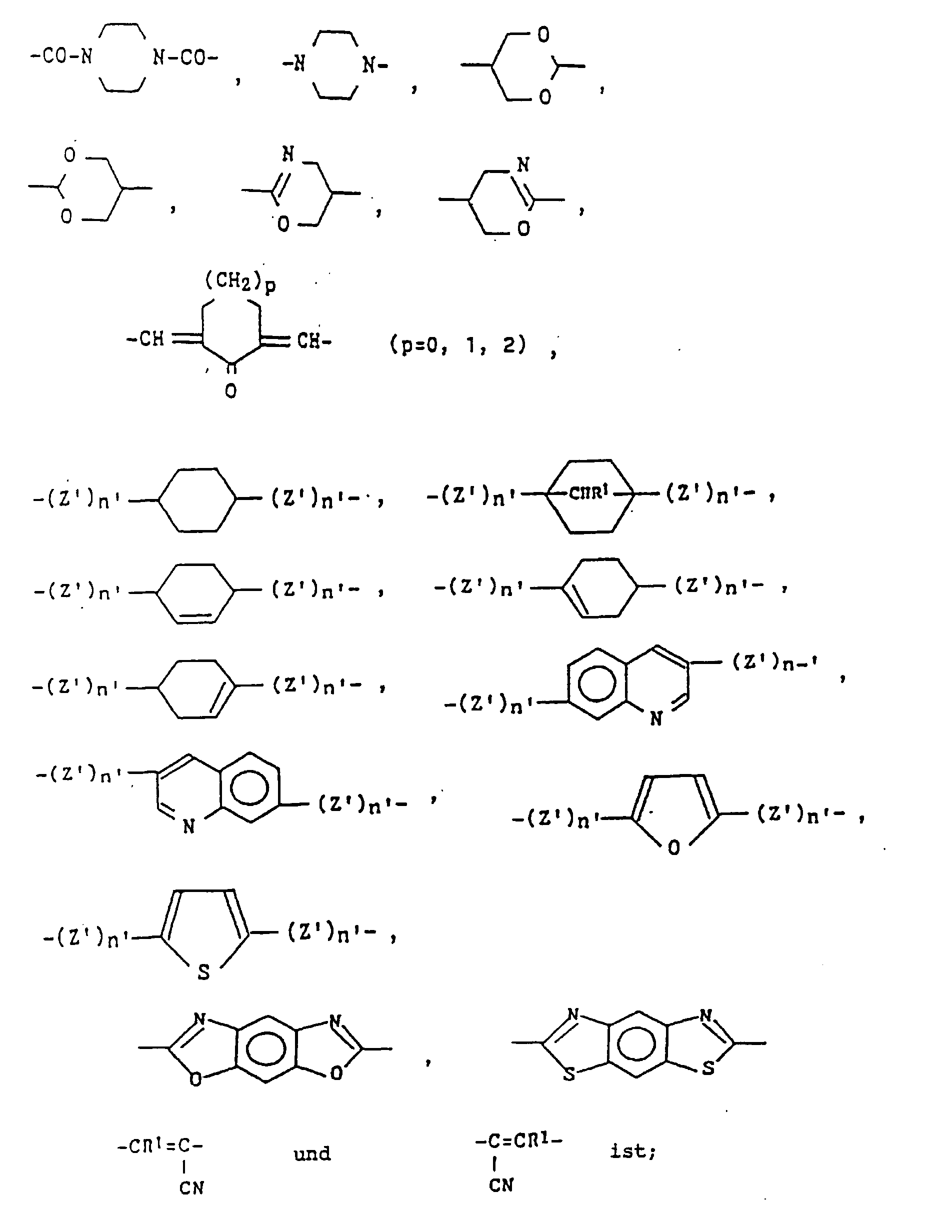 Z² eine Gruppe ist, die durch ein cyclisches oder bicyclisches Ringsystem mit 5 bis 12 Kohlenstoffatomen wiedergegeben wird, oder ein Monoepoxid, welches durch die folgende Formel IV wiedergegeben wird: 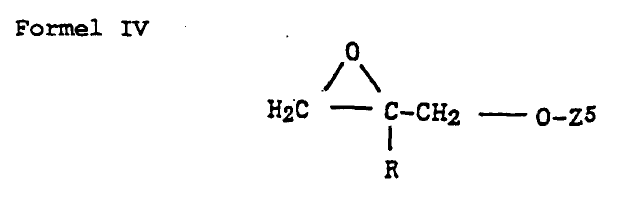 worin Z⁵ ist 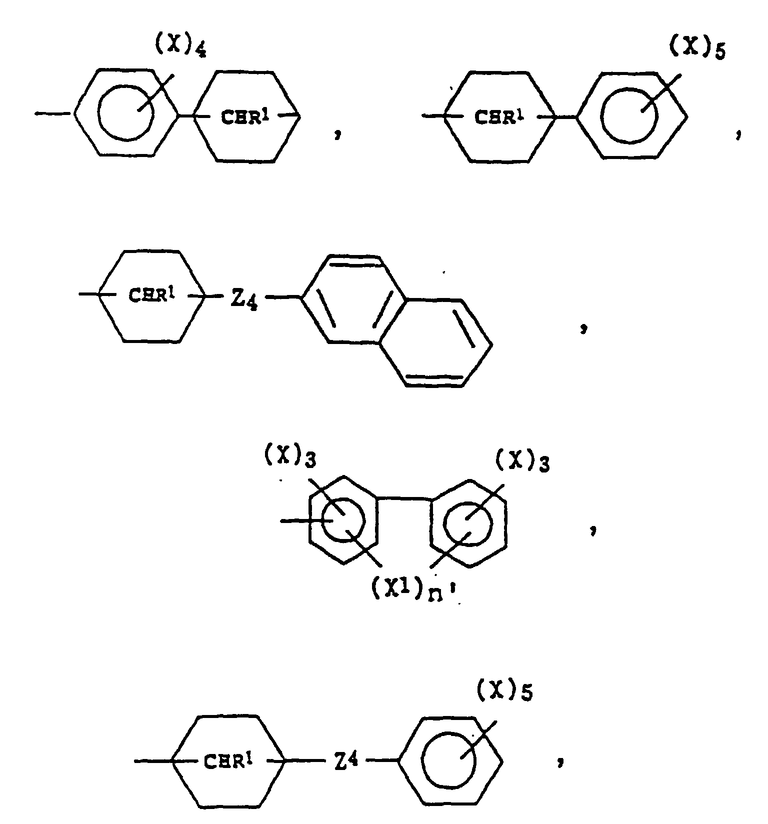 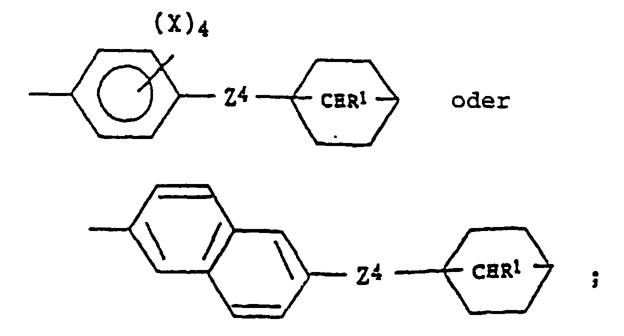 Z⁴ -CO-O-, -O-CO-, -NR¹-CO- oder -CO-NR¹- ist, und
(C) eine aushärtende Menge wenigstens eines Härtere hierfür, worin die Komponente (A) in einer Menge von 1 bis 99 Gew.-% , bezogen auf das kombinierte Gewicht der Komponenten (A) und (B), vorhanden ist, und die Komponente (B) in einer Menge von 99 bis 1 Gew.-%, bezogen auf das kombinierte Gewicht der Komponenten (A) und (B), vorhanden ist.

13. Produkt, resultierend aus dem Aushärten einer der Zusammensetzungen der Ansprüche 11 oder 12.

14. Produkt nach Anspruch 13, resultierend aus dem Anlegen eines elektrischen Feldes, eines magnetischen Feldes oder einer Scherspannung vor dem und/oder während des Aushärten/s.

## Revendications

1. Résine époxyde contenant un ou plusieurs fragments mésogènes ou en forme de bâtonnet, représentée par la formule I suivante : Formule I 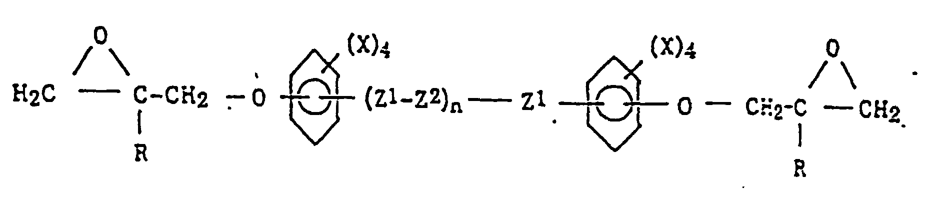 dans laquelle
les groupes de type éther de glycidyle et les raccords de formule -(Z¹-Z²)ₙ-Z¹- se trouvent, en une proportion d'au moins environ 80 %, en position para les uns par rapport aux autres ;
chaque symbole R ou R¹ représente, indépendamment, un atome d'hydrogène ou un groupe hydrocarboné aliphatique comportant de 1 à 4 atomes de carbone ;
chaque symbole X représente, indépendamment, un atome d'hydrogène, un groupe hydrocarbyle ou hydrocarbyl-oxy comportant de 1 à 12 atones de carbone, un atome d'halogène, ou un groupe nitro -NO₂ ou cyano -C≡N ;
chaque symbole Z¹ représente, indépendamment, un groupe de formule -CR¹=CR¹-, -CR¹=CR¹-CR¹=CR¹-, -CR¹=N-N=CR¹-, -CR²=CR¹-CO-O-CH₂-, -CR¹=CR¹-CO-O-CH₂-CH₂-, -CH₂-O-CO-CR¹=CR¹-, -CH₂-CH₂-O-CO-CR¹=CR¹-, -CR¹=CR¹-CO-O-, -O-CO-CR¹=CR¹-, -CO-NH-, -NH-CO-, -CO-NH-NH-CO-, -C≡C-, -C≡C-C≡C-, -CO-S- ou -S-CO-, une liaison simple directe si n ≧ 1, ou un groupe de formule 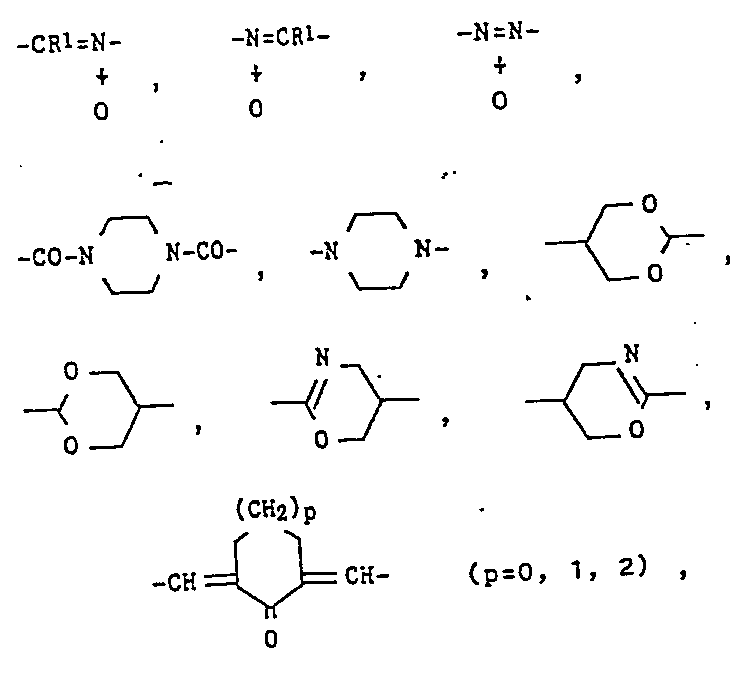 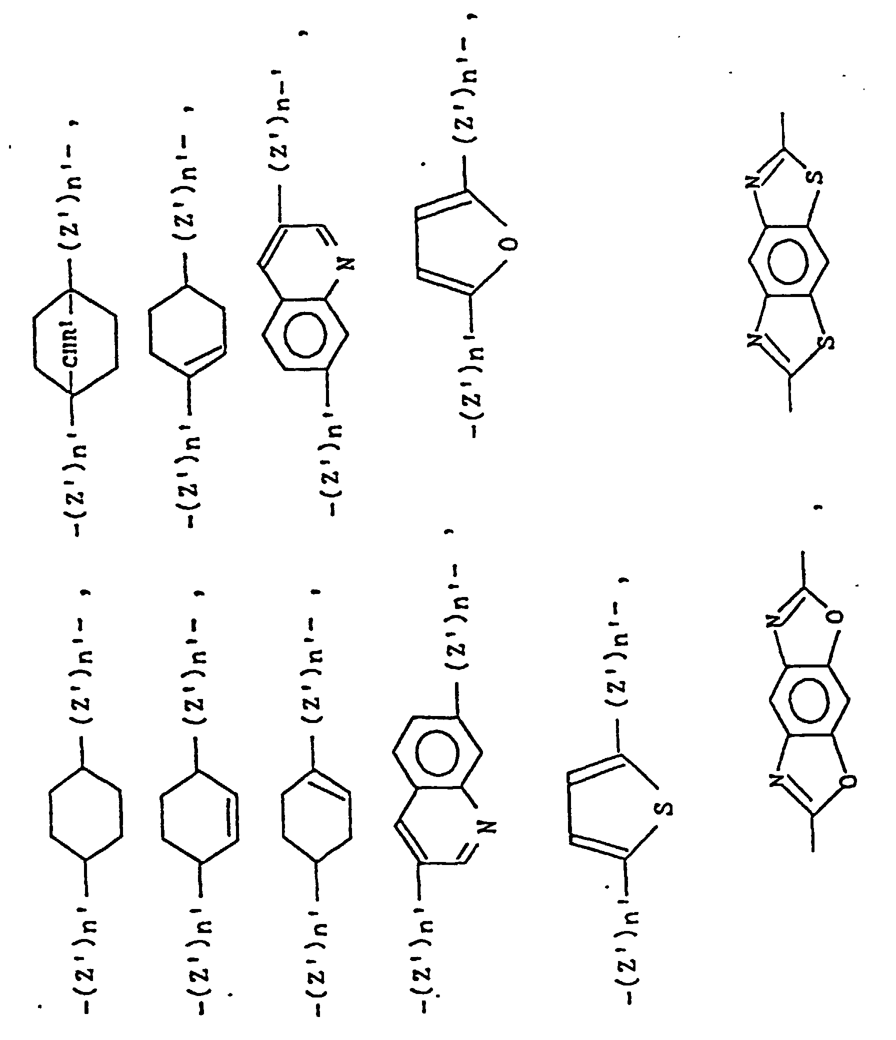 et chaque symbole Z¹ peut également représenter, indépendamment, un groupe de formule 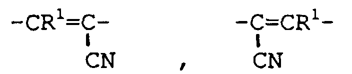 -CR¹=N-, -N=CR¹-, -CO-CR¹=CR¹-, -CR¹=CR¹-CO-, -CO-O- ou -O-CO-, si Z² ne représente pas un cycle benzénique et si n ne vaut pas 0 ;
Z² représente un groupe constitué d'un système cyclique ou bicyclique qui comporte de 5 à 12 atomes de carbone et peut être cycloaliphatique, polycycloaliphatique, aromatique ou une combinaison de ces types ;
n vaut de 0 à 2 ;
chaque symbole Z' représente, indépendamment, un groupe de formule -CO-, -O-CO-, -CO-O-, -CO-NR¹- ou -NR¹-CO- ; et
chaque symbole n' représente indépendamment un nombre qui vaut 0 ou 1,
ou représentée par la formule II suivante : 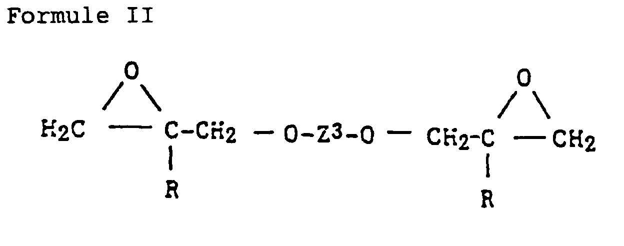 dans laquelle
Z³ représente un groupe de formule 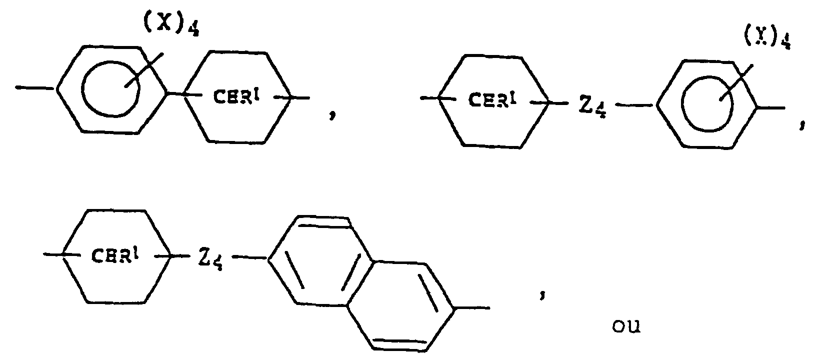 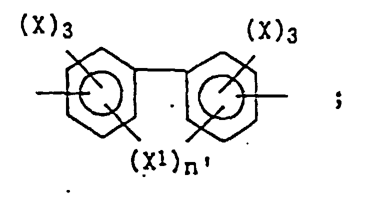 où Z⁴ représente un groupe de formule -CO-O-, -O-CO-, -NR¹-CO- ou -CO-NR¹- ;
X¹ représente un groupe hydrocarbyle qui comporte de 1 à 10 atomes de carbone, qui peut contenir un ou plusieurs hétéroatomes choisis parmi N, O et S, et qui peut être saturé ou insaturé ; et
X, R, R¹ et n' ont les significations indiquées précédemment.

2. Ether diglycidylique du 4,4'-dihydroxy-α-méthylstilbène.

3. Ether diglycidylique du 4,4'-dihydroxybenzanilide.

4. Ether diglycidylique du 4,4'-dihydroxy-2,2'-diméthylazoxybenzène.

5. Composition de résine époxyde prépolymérisée, qu'on prépare en faisant réagir (A) une ou plusieurs des compositions ou résines époxydes conformes à l'une des revendications 1, 2, 3 et 4, avec (B) au moins un composé comportant en moyenne plus d'un atome d'hydrogène actif par molécule, les composants (A) et (B) étant utilisés en des quantités telles que le rapport du nombre d'atomes d'hydrogène actif au nombre de groupes époxydes vaut de 0,01/1 à 0,95/1.

6. Composition de résine thermoplastique, préparée par une réaction de prépolymérisation de (A) une ou plusieurs des compositions ou résines époxydes conformes à l'une des revendications 1, 2, 3, 4 et 5, avec (B) au moins un composé comportant en moyenne plus d'un atome d'hydrogène actif par molécule, les composants (A) et (B) étant utilisés en des quantités telles que le rapport du nombre d'atomes d'hydrogène actif au nombre de groupes époxydes vaut de 0,96/1 à 1,05/1.

7. Composition de résine époxyde conforme à l'une des revendications 1, 2, 3, 4, 5 et 6, dans laquelle les fragments mésogènes ou en forme de bâtonnet sont orientés.

8. Composition de résine époxyde conforme à la revendication 7, dans laquelle ladite orientation est réalisée à l'aide d'un champ électrique, d'un champ magnétique ou d'une contrainte de cisaillement.

9. Mélange comprenant (A) un ou plusieurs composés ou résines comportant des groupes époxydes, comportant un ou plusieurs fragments mésogènes ou en forme de bâtonnet, conformes à l'une des revendications 1, 2, 3 et 4, et (B) un ou plusieurs polyépoxydes ne comportant pratiquement pas de fragments mésogènes ou en forme de bâtonnet, le composant (A) se trouvant en une quantité représentant de 1 à 99 % du poids total des composants (A) et (B), et le composant (B) se trouvant en une quantité représentant de 99 à 1 % du poids total des composants (A) et (B).

10. Mélange comprenant (A) un ou plusieurs composés ou résines comportant des groupes époxydes, comportant un ou plusieurs fragments mésogènes ou en forme de bâtonnet, conformes à la revendication 5, et (B) un ou plusieurs polyépoxydes ne comportant pas de fragments mésogènes ou en forme de bâtonnet, le composant (A) se trouvant en une quantité représentant de 1 à 99 % du poids total des composants (A) et (B), et le composant (B) se trouvant en une quantité représentant de 99 à 1 % du poids total des composants (A) et (B).

11. Composition durcissable comprenant un ou plusieurs des résines époxydes, compositions ou mélanges conformes à l'une des revendications 1 à 5, 9 et 10, et au moins un agent durcisseur en une quantité qui permet de la faire durcir.

12. Composition durcissable comprenant (A) une ou plusieurs des résines époxydes ou compositions conformes à l'une des revendications 1 à 5, (B) un ou plusieurs des composants suivants :
un monoépoxyde contenant un ou plusieurs fragments mésogènes ou en forme de bâtonnet, ce monoépoxyde étant représenté par la formule suivante III : 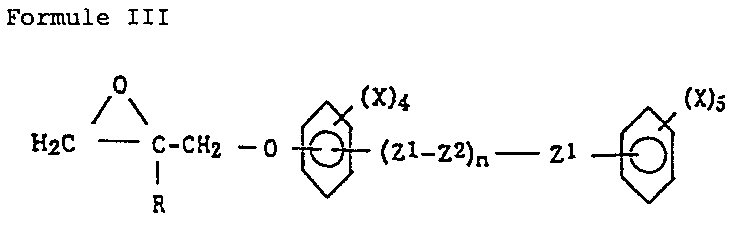 dans laquelle
les groupes de type éther de glycidyle et les raccords de formule -(Z¹-Z²)ₙ-Z¹- se trouvent, en une proportion d'au moins environ 80 %, en position para les uns par rapport aux autres ;
chaque symbole R ou R¹ représente, indépendamment, un atome d'hydrogène ou un groupe hydrocarboné aliphatique comportant de 1 à 4 atomes de carbone ;
chaque symbole X représente, indépendamment, un atome d'hydrogène, un groupe hydrocarbyle ou hydrocarbyl-oxy comportant de 1 à 12 atomes de carbone, un atome d'halogène, ou un groupe nitro -NO₂ ou cyano -C≡N ;
chaque symbole Z¹ représente, indépendamment, un groupe de formule -CR¹=CR¹-, -CR¹=CR¹-CR¹=CR¹-, -CR¹=N-N=CR¹-, -CR¹=CR¹-CO-O-CH₂-, -CR¹=CR¹-CO-O-CH₂-CH₂-, -CH₂-O-CO-CR¹=CR¹-, -CH₂-CH₂-O-CO-CR¹=CR¹-, -CR¹=CR¹-CO-O-, -O-CO-CR¹=CR¹-, -N=N-, -CO-NH-, -NH-CO-, -CO-NH-NH-CO-, -C≡C-, -C≡C-C≡C-, -CO-S-, -CO-NH-, -NH-CO-, -CO-NH-NH-CO-, -C≡C-, -C≡C-C≡C-, -CO-S-, -S-CO-, -CR¹=N-, -N=CR¹-, -CO-O- ou -O-CO-, une liaison simple directe si n ≧ 1, ou un groupe de formule 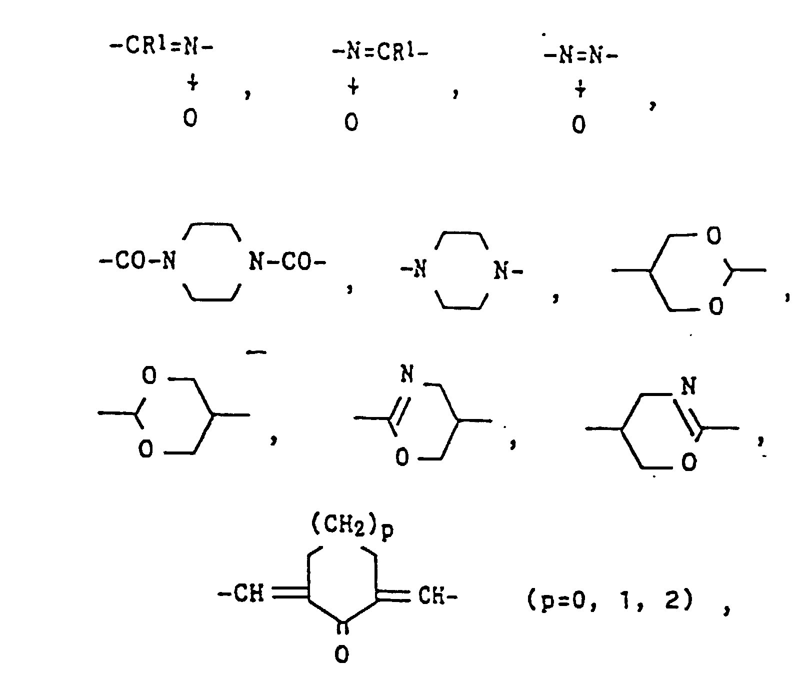 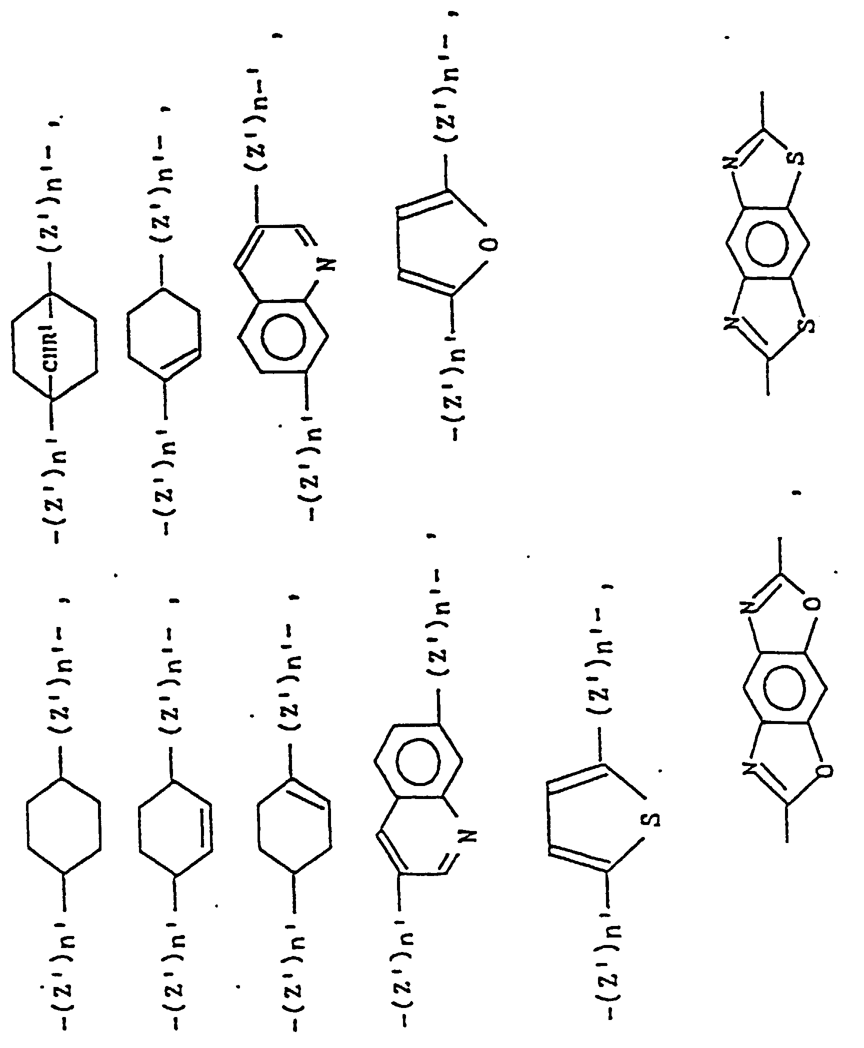 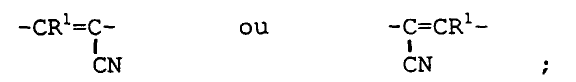 Z² représente un groupe qui est un système cyclique ou bicyclique comportant de 5 à 12 atomes de carbone ;
un monoépoxyde représenté par la formule suivante IV : 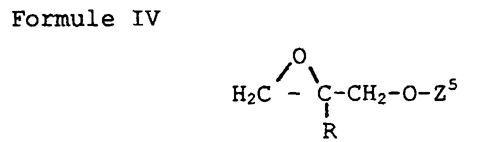 dans laquelle Z⁵ représente 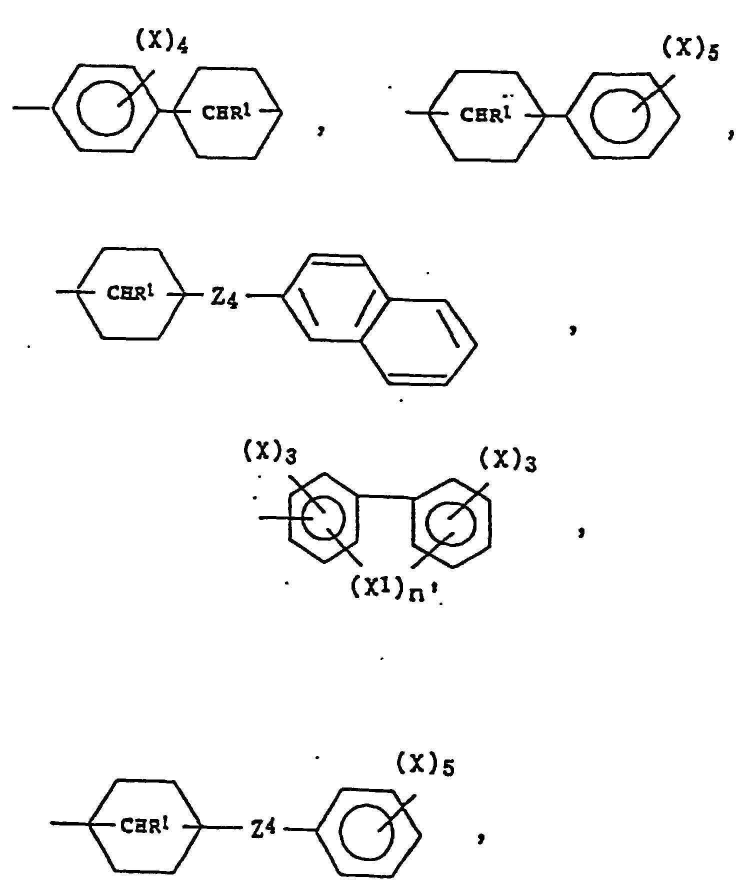 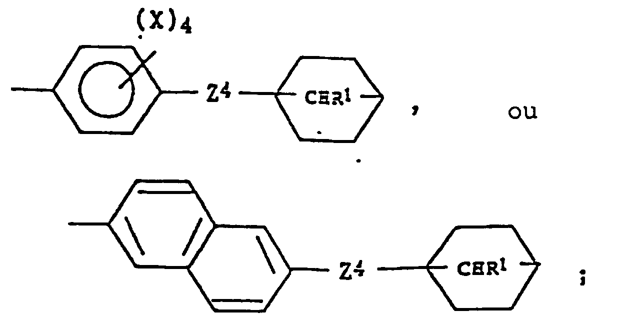 et Z⁴ représente -CO-O-, -O-CO-, -NR¹-CO- ou -CO-NR¹-, et (C) un agent durcissable approprié en une quantité suffisante pour qu'il joue son rôle ;
dans ces mélanges, le composant (A) se trouve en une quantité qui représente de 1 à 99 % du poids total des composants (A) et (B), et le composant (B) se trouve en une quantité qui représente de 99 à 1 % du poids total des composants (A) et (B).

13. Produit résultant du durcissement d'une composition conforme à l'une des revendications 11 et 12.

14. Produit conforme à la revendication 13, résultant de l'application d'un champ électrique, d'un champ magnétique ou d'une contrainte de cisaillement avant et/ou pendant le durcissement.
